(19) 

Europäisches
Patentamt

European
Patent Office

Office européen
des brevets

(11) **EP 4 397 978 A1**

(12) **EUROPEAN PATENT APPLICATION**

published in accordance with Art. 153(4) EPC

(43) Date of publication:
**10.07.2024 Bulletin 2024/28**

(21) Application number: **22864560.2**

(22) Date of filing: **30.08.2022**

(51) International Patent Classification (IPC):
***G01N 33/86*** (2006.01)

(52) Cooperative Patent Classification (CPC):
**G01N 33/86**

(86) International application number:
**PCT/JP2022/032578**

(87) International publication number:
**WO 2023/032978 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2021 JP 2021141790**

(71) Applicant: **Sekisui Medical Co., Ltd.
Tokyo 103-0027 (JP)**

(72) Inventors:
- **KAWABE, Toshiki
  Tokyo 103-0027 (JP)**
- **ONISHI, Kengo
  Tokyo 103-0027 (JP)**

(74) Representative: **Wächtershäuser & Hartz
Patentanwaltspartnerschaft mbB
Weinstraße 8
80333 München (DE)**

(54) **METHOD FOR DETECTING ANOMALY IN BLOOD COAGULATION REACTION**

(57) A method for detecting an anomaly in a blood coagulation reaction, including: 1) detecting a coagulation reaction end point Pe in a coagulation reaction curve of a subject blood specimen; 2) calculating T(X), where T(X) represents a measurement point or time at which the coagulation reaction curve reaches X% of Pe and X denotes a variable larger than 0 and equal to or smaller than 100; and 3) detecting the anomaly in the blood coagulation reaction of the subject blood specimen based on T(X).

EP 4 397 978 A1

**Description**

Field of the Invention

The present invention relates to a method for detecting an anomaly in a blood coagulation reaction.

Background of the Invention

A blood coagulation test is a test for diagnosing a blood coagulation ability of a subject by studying a coagulation reaction of a blood specimen of the subject. In a blood coagulation test, generally, after adding a predetermined reagent to a blood specimen, a coagulation reaction of the blood specimen is measured over time and a blood coagulation time is measured based on an obtained coagulation reaction. Conventionally, the blood coagulation time is used as an index for detecting an anomaly in the blood coagulation ability. Typical examples of the blood coagulation time include a prothrombin time (PT), an activated partial thromboplastin time (APTT), and a thrombin time. In addition, when testing a fibrinogen concentration in accordance with a thrombin time method, a coagulation time from adding a certain amount of thrombin to a diluted plasma specimen to deposition of fibrin may be measured. In measurement of a coagulation reaction, generally, optical means which measures a scattered light quantity, transmittance, absorbance, or the like, mechanical means which measures a viscosity of blood plasma, or the like is used. More recently, automated analyzers for coagulation reaction measurement are being widely used.

A blood coagulation reaction may contain various noises attributable to states of a reaction measuring unit, a reagent, a specimen, and the like. For example, a phenomenon called an early response anomaly in which a coagulation reaction curve rises before the onset of a true coagulation reaction may occur. In addition, a coagulation reaction curve may acquire an abnormal shape due to a disorder of the blood coagulation ability, an effect of medication, or the like. Such an occurrence of an anomaly in the coagulation reaction curve constitutes a hindrance to accurate measurement of a coagulation time.

There is a need to correctly assess a coagulation reaction by eliminating noise and other adverse effects from the coagulation reaction. Patent Literature 1 describes a method of analyzing a blood coagulation reaction in which an early response anomaly is detected by providing a check point or a check region between a start of measurement of the blood coagulation reaction and an end of the coagulation reaction and monitoring a state of reaction at the check point or in the check region. Patent Literature 2 describes preventing an erroneous detection of a coagulation time due to noise by setting a noise determination line based on a relationship between a coagulation time and a scattered light quantity measured at the coagulation time, determining a coagulation time of a subject specimen to be normal when a scattered light quantity at the coagulation time of the subject specimen exceeds the noise determination line, and when the scattered light quantity at the coagulation time of the subject specimen does not exceed the noise determination line, continuing measurement of a coagulation reaction until a normal coagulation time is acquired. Patent Literature 3 describes determining an anomaly in a coagulation reaction based on a ratio of a difference between a time ($T[i]$) corresponding to a coagulation rate ($i$) and a time ($T[i + 1]$) corresponding to a coagulation rate ($i + 1$), ($T[i+1] - T[i]$), to a time (SP to EP) from a coagulation start point to a coagulation end point.

In conventional blood coagulation tests, generally, a coagulation reaction is measured at a time sufficient to ensure the coagulation reaction is completed and a coagulation time is calculated based on acquired data. However, blood specimens which contain an anomaly in a coagulation reaction often exhibit a delay in a start or a progress of the coagulation reaction and, consequently, more time tends to be required for the coagulation reaction to end. Therefore, particularly when a coagulation reaction is measured with an automated analyzer, a measurement time must be set longer in order to avoid missing out on data, but setting a longer measurement time means that a longer time is required to obtain a test result and overall analysis efficiency declines.

Patent Literature 1: JP-A-2003-169700
Patent Literature 2: JP-A-2010-217059
Patent Literature 3: JP-A-2007-107889

Summary of the Invention

The present invention provides a method of detecting an anomaly in a blood coagulation reaction attributable to noise or the like.

Specifically, the present invention provides the following.

[1] A method for detecting an anomaly in a blood coagulation reaction, comprising:

1) detecting a coagulation reaction end point Pe in a coagulation reaction curve of a subject blood specimen;
2) calculating T(X), wherein T(X) represents a measurement point or time at which the coagulation reaction curve reaches X% of Pe and X denotes a variable larger than 0 and equal to or smaller than 100; and
3) detecting the anomaly in the blood coagulation reaction of the subject blood specimen based on T(X).

[2] The method according to [1], wherein 3) comprises calculating an index R based on T(X) and detecting presence or absence of the anomaly in the blood coagulation reaction of the subject blood specimen based on the index R.
[3] The method according to [2], wherein the index R is at least one selected from the group consisting of a ratio of T(X) in a given range of X, a rate of change of T(X) in a given range of X, and a coefficient of variation of T(X) in a given range of X.
[4] The method according to [3], wherein:

the ratio of T(X) in the given range of X is $[T(X_1)/T(X_2)]$;
the rate of change of T(X) in the given range of X is $[(T(X_3) - T(X_5))/T(X_4)]$; and
the coefficient of variation of T(X) in the given range of X is a coefficient of variation of a coefficient of variation T(X) in a range from $X_6$ to $X_7$.

[5] The method according to any one of [2] to [4], comprising detecting presence or absence of the anomaly in the blood coagulation reaction of the subject blood specimen by comparing the index R with a given threshold.
[6] The method according to [5], wherein the threshold is determined based on the index R acquired from a group of specimens.
[7] The method according to [5], wherein the anomaly in the blood coagulation reaction of the subject blood specimen is detected when:

the threshold is equal to or larger than "mean + 2 × standard deviation" of index R acquired from a group of normal specimens, and the index R of the subject blood specimen is equal to or larger than the threshold, or
the threshold is equal to or smaller than "mean - 2 × standard deviation" of index R acquired from a group of normal specimens, and the index R of the subject blood specimen is equal to or smaller than the threshold.

[8] The method according to [1], wherein 3) comprises calculating indexes R1 and R2 based on T(X) and detecting a type of the anomaly in the blood coagulation reaction of the subject blood specimen based on the indexes R1 and R2 .
[9] The method according to [8], wherein

the index R1 is a rate of change of T(X) in a first given range of X, and
the index R2 is
a rate of change of T(X) in a second given range of X or a coefficient of variation of T(X) in a given range of X.

[10] The method according to [9], wherein

the rate of change of T(X) in the first given range of X is $[(T(X_1) - T(X_3))/T(X_2)]$;
the rate of change of T(X) in the second given range of X is $[(T(X_4) - T(X_6))/T(X_5)]$ ; and
the coefficient of variation of T(X) in the given range of X is a coefficient of variation of T(X) in a range from $X_7$ to $X_8$.

[11] The method according to [9] or [10], comprising

comparing R1 with a threshold Th1 and comparing R2 with a threshold Th2, and
detecting the type of the anomaly in the blood coagulation reaction of the subject blood specimen based on whether or not R1 and R2 had reached the respective thresholds, wherein
R1 is the rate of change of T(X) in the first given range of X and R2 is the rate of change of T(X) in the second given range of X.

[12] The method according to [9] or [10], comprising

comparing R1 with a threshold Th1 and, when R1 had reached Th1, detecting that the subject blood specimen has the anomaly in the blood coagulation reaction,
comparing R2 of the subject blood specimen having been detected to have the anomaly in the blood coagulation reaction with thresholds Th2 and Th3, and

detecting the type of the anomaly in the blood coagulation reaction of the subject blood specimen based on whether or not R2 had respectively reached Th2 and Th3, wherein
R1 is the rate of change of T(X) in the first given range of X and R2 is the coefficient of variation of T(X) in the given range of X.

[13] The method according to [11], wherein the threshold Th1 is determined based on the index R1 acquired from a group of specimens and the threshold Th2 is determined based on the index R2 acquired from the group of specimens.

[14] The method according to [12], wherein the threshold Th1 is determined based on the index R1 acquired from a group of specimens and the thresholds Th2 and Th3 are determined based on the index R2 acquired from the group of specimens.

[15] The method according to any one of [1] to [14], further comprising calculating a coagulation time of the subject blood specimen.

[16] The method according to any one of [1] to [15], wherein the detection of Pe is performed in parallel to acquisition of the coagulation reaction curve of the subject blood specimen, and the acquisition of the coagulation reaction curve is ended once Pe is detected.

[17] A program for implementing the method according to any one of [1] to [16].

[18] An apparatus for implementing the method according to any one of [1] to [16].

Advantageous Effects of the Invention

According to the present invention, an anomaly in a blood coagulation reaction attributable to noise or the like can be detected. Therefore, according to the present invention, an erroneous measurement of a blood coagulation time or an erroneous assessment of a blood coagulation ability of a subject attributable to an anomaly in a blood coagulation reaction can be prevented.

Brief Description of Drawings

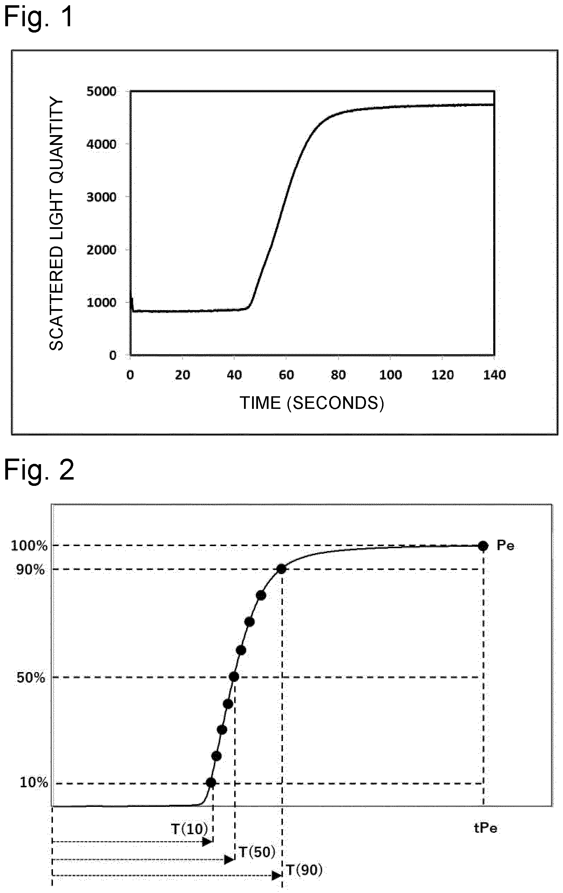

[Figure 1] Figure 1 shows an example of a coagulation reaction curve.

[Figure 2] Figure 2 is an illustration of T(X). Time points on a coagulation reaction curve where 10%, 50%, and 90% of Pe are reached are respectively denoted as T(10), T(50), and T(90).

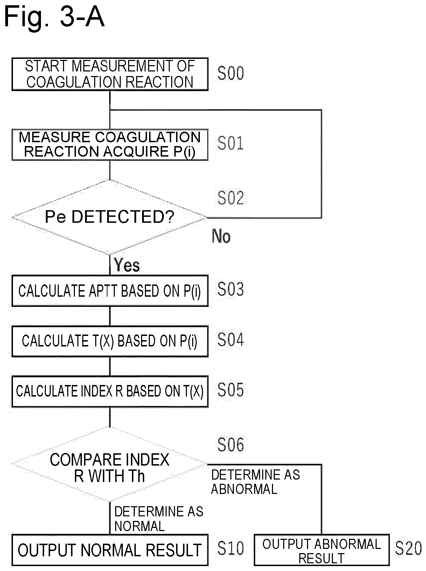

[Figure 3-A] Figure 3-A is an exemplary flow for detecting an anomaly in a coagulation reaction by a method of the present invention.

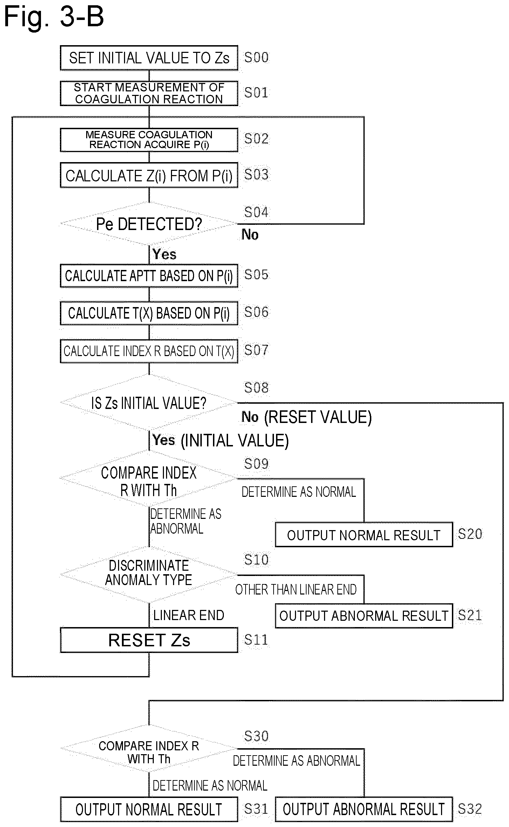

[Figure 3-B] Figure 3-B is an exemplary flow for detecting an anomaly in a coagulation reaction by the method of the present invention when detection of Pe is performed using an integrated ratio Z(i) of P(i) and a threshold Zs.

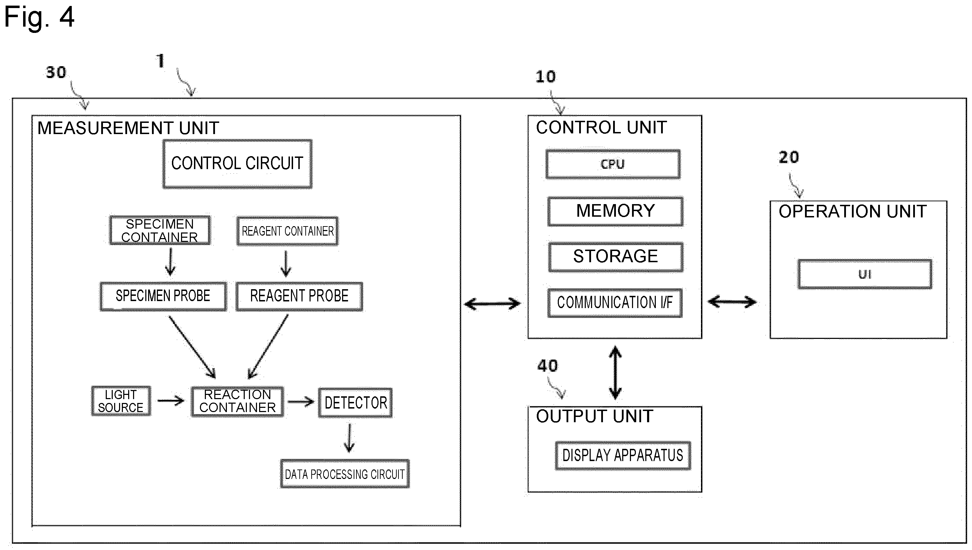

[Figure 4] Figure 4 is a conceptual diagram showing a configuration of an automated analyzer for carrying out a method for detecting an anomaly in a coagulation reaction according to the present invention.

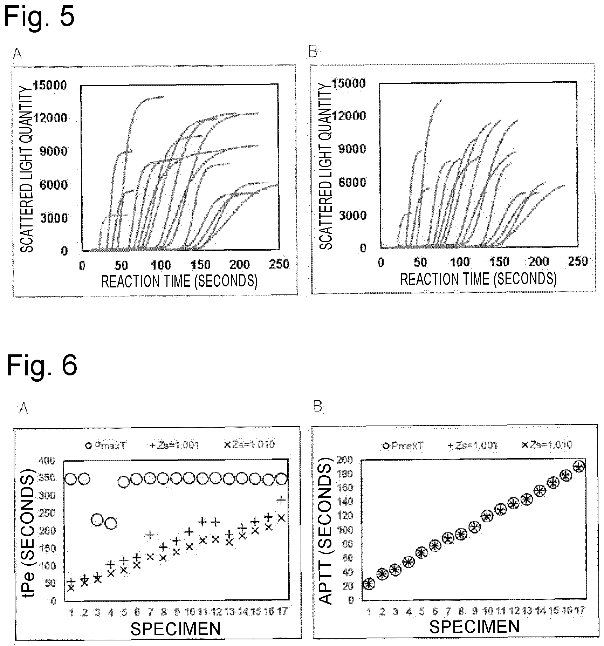

[Figure 5] Figure 5 shows a reaction curve P (scattered light quantity) of a normal specimen group (specimens 1 to 17). A shows a reaction curve P up to a coagulation reaction end point Pe obtained when Zs = 1.001 is set, and B shows the reaction curve P up to the coagulation reaction end point Pe obtained when Zs = 1.010 is set.

[Figure 6] Figure 6 shows, in A, a time tPe at which the reaction curve P of the normal specimen group (specimens 1 to 17) reaches Pe, and in B, APTT with respect to the specimens 1 to 17. PmaxT (○) denotes data when a maximum value Pmax of the reaction curve P is adopted as Pe, Zs = 1.001 (+) denotes data upon detecting Pe when Zs = 1.001 is set, and Zs = 1.010 (×) denotes data upon detecting Pe when Zs = 1.010 is set.

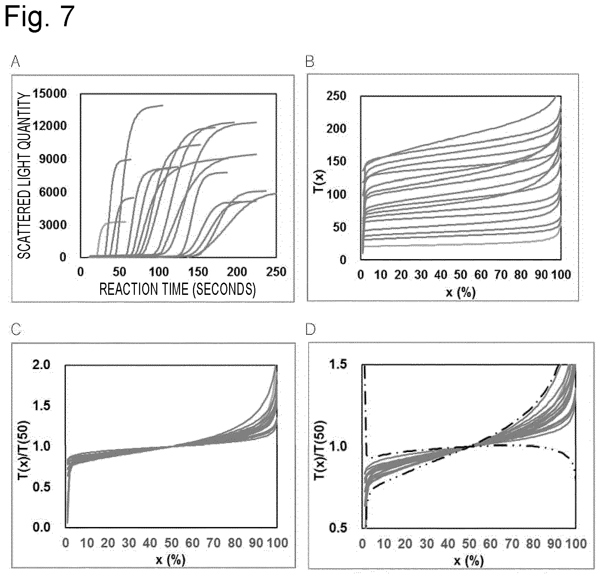

[Figure 7] Figure 7 shows a coagulation reaction of the normal specimen group (specimens 1 to 17) based on Pe detected when Zs = 1.001 is set. A shows the reaction curve P (scattered light quantity) up to Pe, B shows T(X), C shows T(X)/T(50), and D shows an enlarged view of C in a direction of an axis of ordinate, where a dashed-dotted line represents mean + 3SD and a dashed-two dotted line represents mean - 3SD.

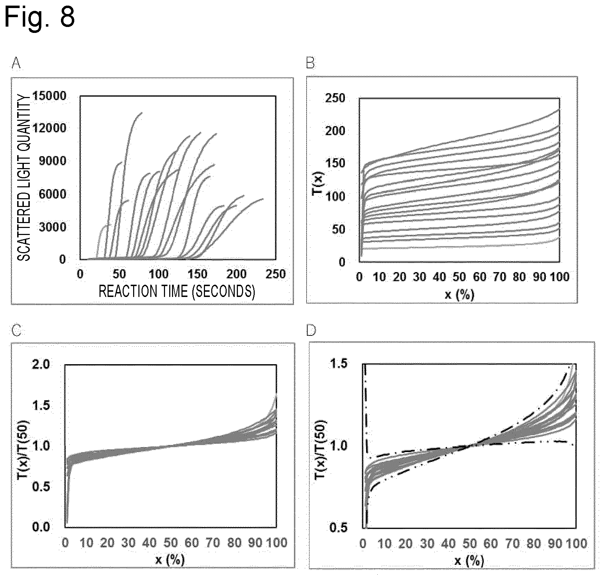

[Figure 8] Figure 8 shows a coagulation reaction of the normal specimen group (specimens 1 to 17) based on Pe detected when Zs = 1.010 is set. A shows the reaction curve P (scattered light quantity) up to Pe, B shows T(X), C shows T(X)/T(50), and D shows an enlarged view of C in a direction of an axis of ordinate, where a dashed-dotted line represents mean + 3SD and a dashed-two dotted line represents mean - 3SD.

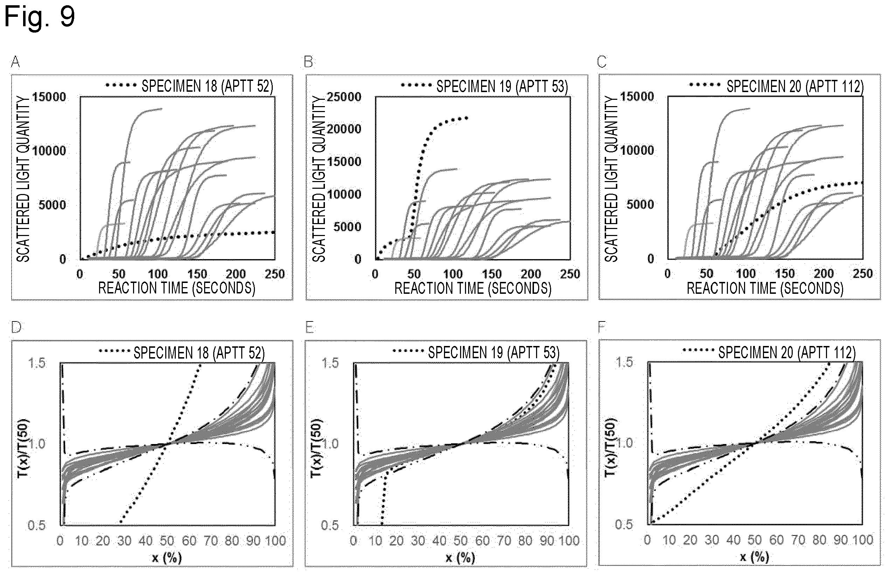

[Figure 9] Figure 9 shows a coagulation reaction of abnormal specimens (specimens 18 to 20) based on Pe detected when Zs = 1.001 is set. A to C show the reaction curve P (scattered light quantity) up to Pe of specimens 18 (A), 19 (B), and 20 (C) together with data of normal specimens, where a solid line represents a normal specimen and a dotted line represents an abnormal specimen. D to F show T(X)/T(50) of specimens 18 (D), 19 (E), and 20 (F) together with data of normal specimens, where a solid line represents a normal specimen, a dotted line represents an abnormal specimen, and a dashed-dotted line and a dashed-two dotted line respectively represent mean + 3SD and mean - 3SD with respect to a normal specimen group.

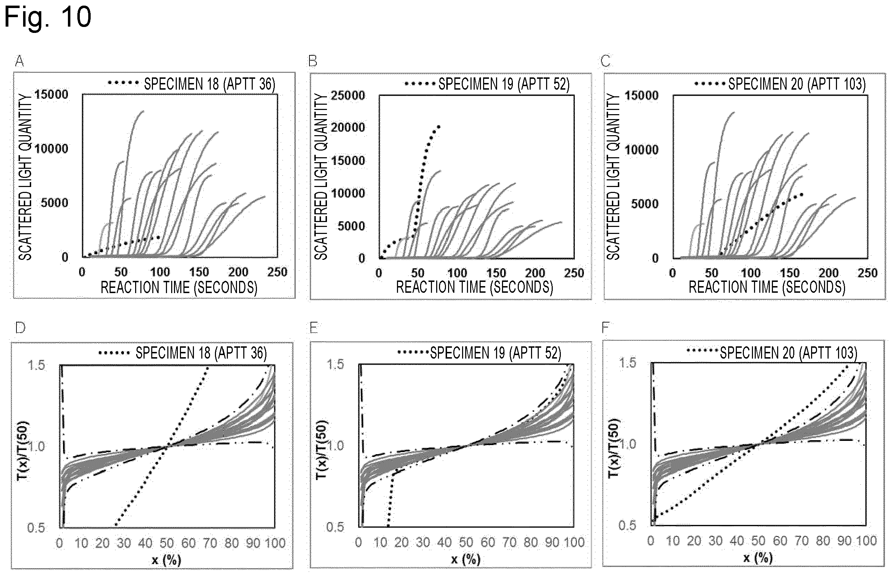

[Figure 10] Figure 10 shows a coagulation reaction of abnormal specimens (specimens 18 to 20) based on Pe detected when Zs = 1.010 is set. A to C show the reaction curve P (scattered light quantity) up to Pe of specimens 18 (A), 19 (B), and 20 (C) together with data of normal specimens, where a solid line represents a normal specimen and a dotted line represents an abnormal specimen. D to F show $T(X)/T(50)$ of specimens 18 (D), 19 (E), and 20 (F) together with data of normal specimens, where a solid line represents a normal specimen, a dotted line represents an abnormal specimen, and a dashed-dotted line and a dashed-two dotted line respectively represent mean + 3SD and mean - 3SD with respect to a normal specimen group.

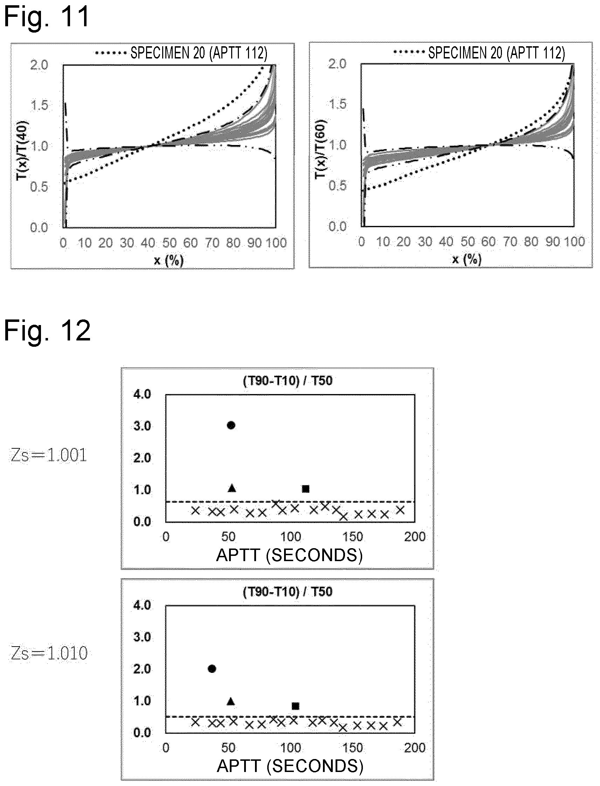

[Figure 11] Figure 11 shows $T(X)/T(40)$ (left) and $T(X)/T(60)$ (right) of an abnormal specimen (specimen 20). A solid line represents a normal specimen, a dotted line represents the abnormal specimen (specimen 20), and a dashed-dotted line and a dashed-two dotted line respectively represent mean + 3SD and mean - 3SD with respect to a normal specimen group.

[Figure 12] Figure 12 shows a plot of a rate of interval change of $T(X)$, $[(T(90) - T(10))/T(50)]$, with respect to APTT. An upper half shows a plot when Zs = 1.001 and a lower half shows a plot when Zs = 1.010, where $\times$ denotes a normal specimen, ● denotes an abnormal specimen (specimen 18), A denotes an abnormal specimen (specimen 19), and ■ denotes an abnormal specimen (specimen 20). A dotted line represents a mean of values of a normal specimen group + 3SD.

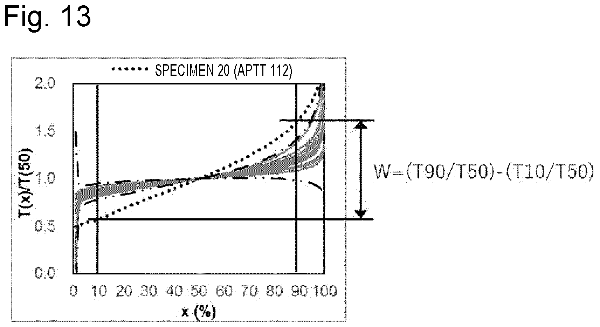

[Figure 13] Figure 13 is an illustration of an index $[(T(90) - T(10))/T(50)]$. The index denotes W $[(T(90)/T(50)) - (T(10)/T(50))]$ in the diagram.

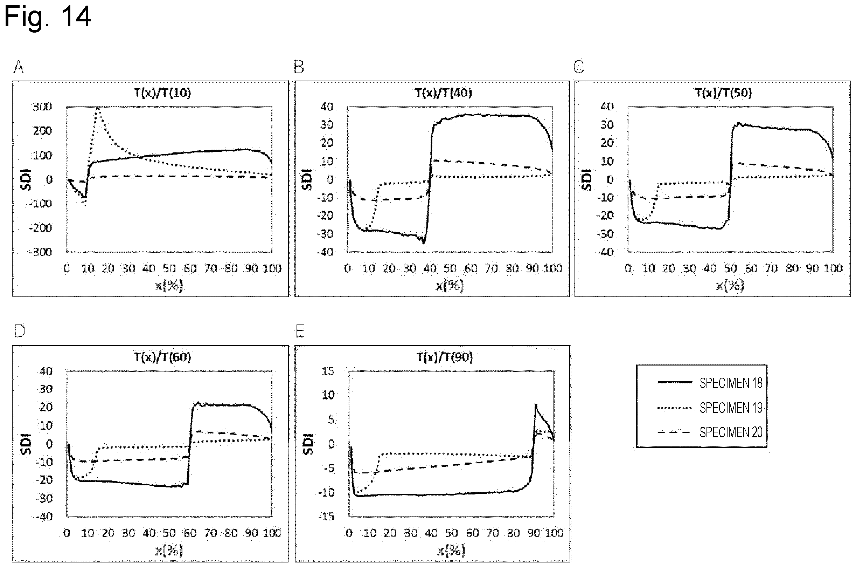

[Figure 14] Figure 14 shows $SDI(X) = [(R(X) - M)/SD]$ of abnormal specimens (specimens 18 to 20). $R(X)$ denotes $T(X)/T(Y)$ (where X ranges from 1 to 100 and Y assumes 10, 40, 50, 60, and 90 in A to E, respectively), and M and SD denote a mean and an SD of $R(X)$ with respect to the normal specimen group (specimens 1 to 17). In the drawings, a solid line represents the specimen 18, a dotted line represents the specimen 19, and a dashed line represents the specimen 20.

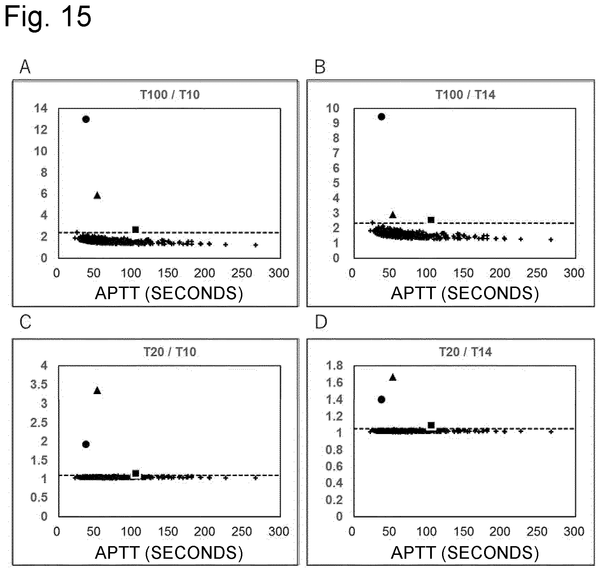

[Figure 15] Figure 15 shows a plot of a ratio of $T(X)$, $[T(X_1)/T(X_2)]$, with respect to APTT. + denotes a control specimen, ● denotes an abnormal specimen (specimen 18), A denotes an abnormal specimen (specimen 19), ■ denotes an abnormal specimen (specimen 20), and a dotted line denotes a mean of values of a control specimen group + 5SD.

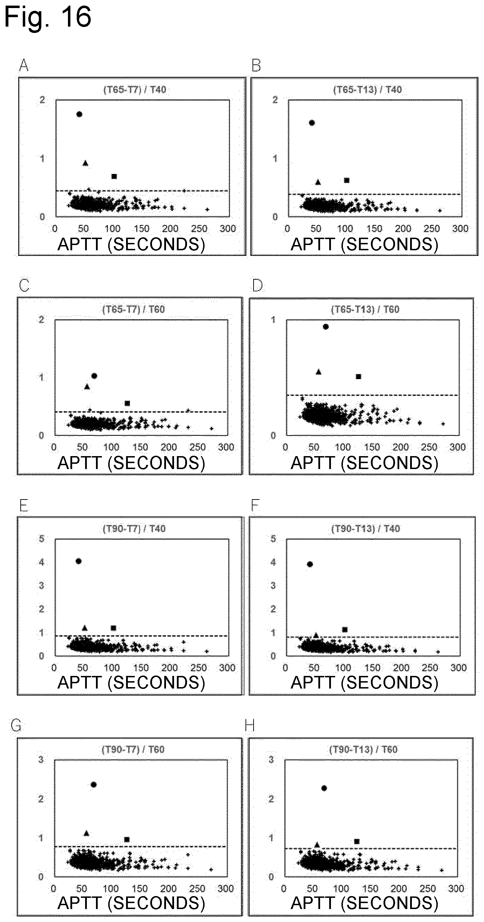

[Figure 16] Figure 16 shows a plot of a rate of interval change of $T(X)$, $[(T(X_1) - T(X_3))/T(X_2)]$, with respect to APTT. + denotes a control specimen, ● denotes an abnormal specimen (specimen 18), A denotes an abnormal specimen (specimen 19), ■ denotes an abnormal specimen (specimen 20), and a dotted line denotes a mean of values of a control specimen group + 5SD.

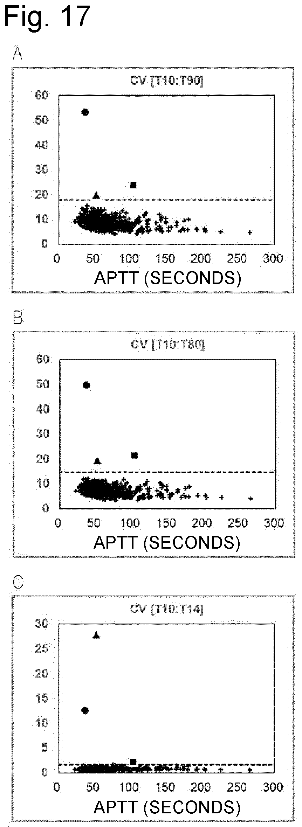

[Figure 17] Figure 17 shows a plot of CV $[T(X_1):T(X_2)]$ with respect to APTT. + denotes a control specimen, ● denotes an abnormal specimen (specimen 18), A denotes an abnormal specimen (specimen 19), ■ denotes an abnormal specimen (specimen 20), and a dotted line denotes a mean of values of a control specimen group + 5SD.

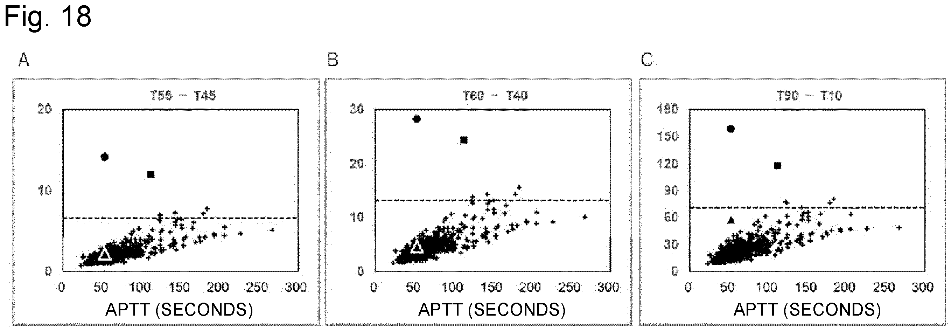

[Figure 18] Figure 18 shows a plot of a difference in $T(X)$, $[T(X_1) - T(X_2)]$, with respect to APTT. + denotes a control specimen, ● denotes an abnormal specimen (specimen 18), A denotes an abnormal specimen (specimen 19), ■ denotes an abnormal specimen (specimen 20), and a dotted line denotes a mean of values of a control specimen group + 5SD.

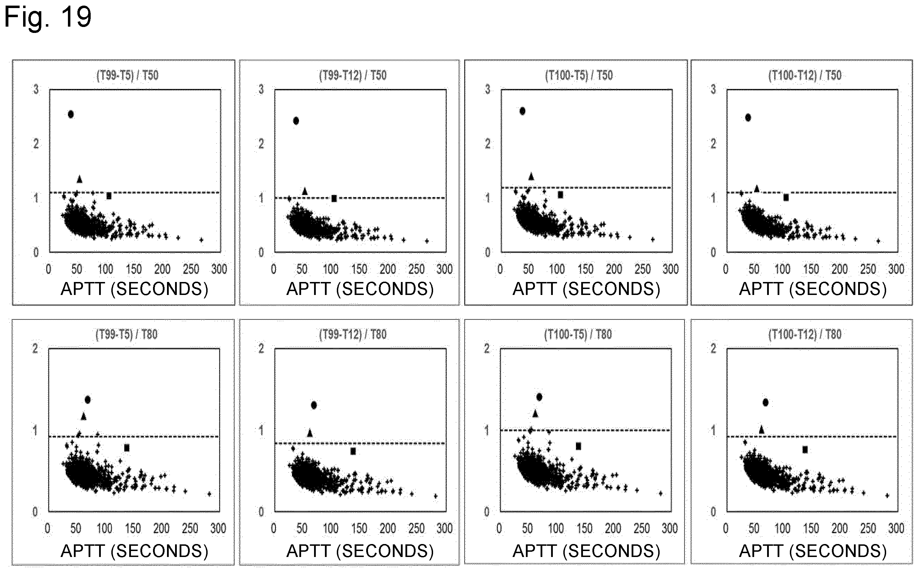

[Figure 19] Figure 19 shows a plot of an example of an index R1 for anomaly type determination, $[(T(X_1)-T(X_3))/T(X_2)]$ (where $X_1$ = 99 to 100, $X_2$ = 50 to 80, and $X_3$ = 5 to 12), with respect to APTT.

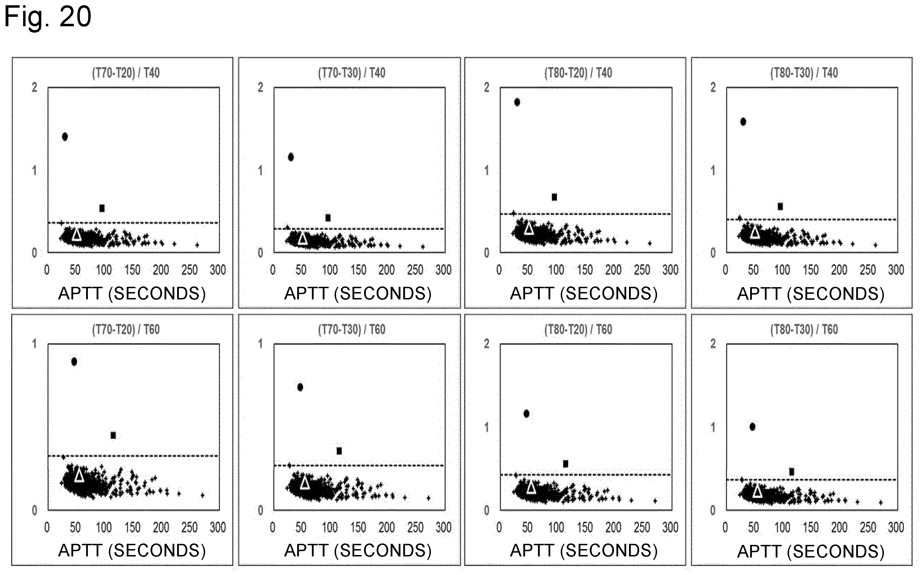

[Figure 20] Figure 20 shows a plot of an example of an index R2 for anomaly type determination, $[(T(X_4)-T(X_6))/T(X_5)]$ (where $X_4$ = 70 to 80, $X_5$ = 40 to 60, and $X_6$ = 20 to 30), with respect to APTT.

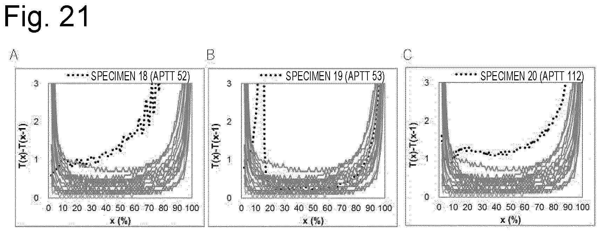

[Figure 21] Figure 21 shows $d(X) = [T(X) - T(X - 1)]$ of A to C, denoting the normal specimen group (specimens 1 to 17) and abnormal specimens (specimens 18 to 20). A solid line denotes a normal specimen, and a dotted line denotes specimens 18 (A), 19 (B), and 20 (C).

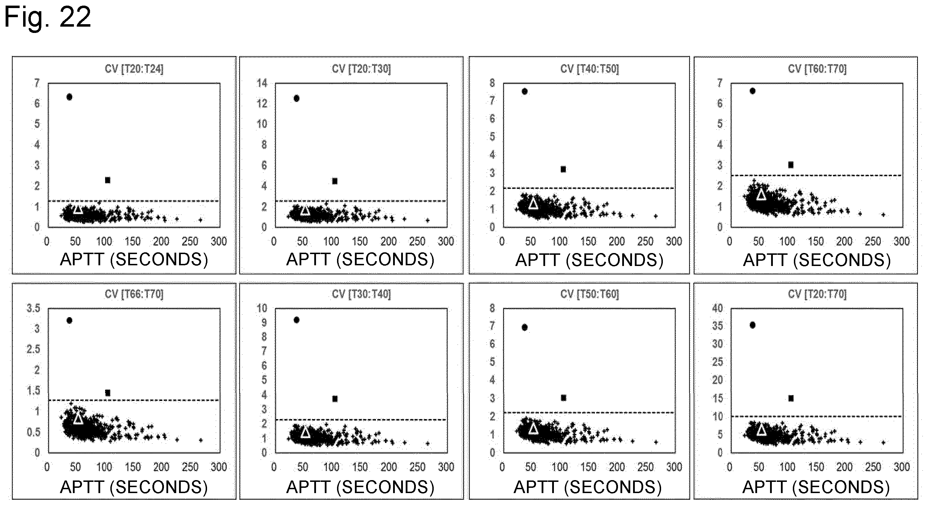

[Figure 22] Figure 22 shows a plot of CV $[T(X_1):T(X_2)]$ with respect to APTT. + denotes a control specimen, ● denotes an abnormal specimen (specimen 18), A denotes an abnormal specimen (specimen 19), ■ denotes an abnormal specimen (specimen 20), and a dotted line denotes a mean of values of a control specimen group + 5SD.

Detailed Description of the Invention

In a blood coagulation test, a predetermined reagent is added to a blood specimen and a subsequent blood coagulation reaction is measured. A blood coagulation reaction is generally represented by a coagulation reaction curve indicating a change in an amount of a coagulation reaction over time. Conventionally, a blood coagulation time measured based on a coagulation reaction is used as an index for detecting an anomaly in the blood coagulation ability. In the present specification, a blood specimen, a blood coagulation reaction, and a blood coagulation time may be simply referred to, respectively, as a specimen, a coagulation reaction, and a coagulation time.

In a case of a measurement based on a scattered light quantity, a coagulation reaction curve usually exhibits a gradual rise as a coagulation reaction starts at a time point where a certain amount of time has elapsed from the addition of a reagent to the specimen, rises abruptly as the coagulation reaction progresses, subsequently reaches a plateau as the coagulation reaction nears its end, and finally exhibits a sigmoidal shape. On the other hand, a coagulation reaction curve measured based on a transmitted light quantity exhibits an inverse sigmoidal shape. However, a coagulation reaction may include noise such as an early response anomaly and such noise may affect a shape of a coagulation reaction curve and constitute a hindrance to accurate measurement of coagulation time. In addition, a coagulation reaction curve of a specimen with a coagulation reaction containing an anomaly exhibits an abnormal shape in which a reaction is smaller, a start or an end of the reaction is unclear, an abrupt rise does not appear, or the like and may make it difficult to accurately measure a coagulation time. Alternatively, the administration of a drug (such as heparin) which affects a blood coagulation ability may also affect the coagulation reaction curve.

In a blood coagulation test by an automated analyzer, it is desirable to complete the measurement as soon as the data necessary for the evaluation of the coagulation reaction of one specimen is obtained and to start the measurement of the next specimen in order to efficiently analyze a large number of specimens. However, when taking the effect of noise and cases of abnormal specimens described above into consideration, such a method carries a risk of missing out on data due to an erroneous detection or a non-detection of the end of the coagulation reaction. Setting a coagulation reaction measurement time per specimen to a sufficiently long time enables missing out on data due to an erroneous detection or a non-detection of the end of the coagulation reaction to be prevented. However, since such methods cause the measurement time to become longer than necessary for many specimens, a longer time is required until a test result is obtained and overall analysis efficiency declines.

According to the present invention, an anomaly in a blood coagulation reaction attributable to noise or the like can be detected by analyzing a shape of a coagulation reaction curve.

Preferably, examples of an "anomaly" in a blood coagulation reaction which is detected in the present invention include an early response anomaly (for example, a premature rise of a coagulation reaction curve before a time at which a true coagulation reaction is supposed to start), a coagulation reaction a start or an end of which is unclear (for example, a coagulation reaction a rise of a coagulation reaction curve of which is unknown or a coagulation reaction a progress of which is slow and a coagulation reaction curve does not plateau), and a coagulation reaction a coagulation reaction curve of which exhibits a biphasic rise.

A "normal" blood coagulation reaction described in the present specification as a contrasting concept preferably refers to a coagulation reaction a coagulation reaction curve of which has a sigmoidal shape (in a case of a measurement based on a "scattered light quantity"). In other words, a normal coagulation reaction curve exhibits a sigmoidal shape which has three intervals following a certain latency from the addition of a coagulation reagent to a specimen of: a "reaction initial stage" in which the curve starts to gradually rise; a "reaction progressing stage" in which the curve abruptly rises and which includes an inflection point; and a "reaction later stage" in which the rise of the curve moderates and plateaus (becomes planarized). Therefore, a typical example of the anomaly in a coagulation reaction detected in the present invention is a coagulation reaction a coagulation reaction curve of which does not have a sigmoidal shape (in a case of a measurement based on a "scattered light quantity").

[Method for detecting anomaly in blood coagulation reaction]

In a method for detecting an anomaly in a blood coagulation reaction provided by the present invention (hereinafter, also referred to as a method of the present invention), presence or absence of an anomaly in a coagulation reaction is detected based on coagulation reaction curve data acquired by measuring a coagulation reaction of a subject blood specimen (hereinafter, also referred to as a subject specimen).

Hereinafter, the method of the present invention will be described.

1. Acquisition of coagulation reaction curve and detection of coagulation reaction end point Pe

1.1. Coagulation reaction measurement

The subject specimen used in the method of the present invention need only be a blood specimen the presence or absence of an anomaly of which in a blood coagulation reaction needs to be detected. As the subject specimen, blood plasma of a subject being tested is preferably used. An anticoagulant normally used in a coagulation test can be added to the subject specimen. For example, blood plasma is obtained by collecting blood using a blood collection tube with sodium citrate and subsequently subjecting the collected blood to centrifugal separation and the obtained blood plasma can be used as the subject specimen.

A blood coagulation reaction measurement with respect to the subject specimen is performed and time-series data of the coagulation reaction is acquired. A coagulation reaction curve of the subject specimen is acquired based on the time-series data. The blood coagulation reaction measurement can be performed in accordance with any method used for a blood coagulation test such as methods used for measuring a coagulation time during a fibrinogen concentration test based on a prothrombin time (PT), an activated partial thromboplastin time (APTT), or a thrombin time method. In the present specification presented below, the method of the present invention will be described mainly in accordance with a method for APTT measurement. Any person skilled in the art can apply the method of the present invention to other methods for measuring a coagulation time (for example, a prothrombin time (PT)).

In a measurement of a blood coagulation reaction, a reagent for coagulation time measurement is added to the subject specimen to start a coagulation reaction. A coagulation reaction of a mixed liquid containing the reagent and the subject specimen can be measured. The reagent for coagulation time measurement to be used can be optionally selected according to a purpose of measurement. Reagents for measuring various coagulation times are commercially available (for example, APTT Reagent Coagpia APTT-N manufactured by SEKISUI MEDICAL CO., LTD.). In the measurement of a coagulation reaction, general means such as optical means which measures a scattered light quantity, transmittance, absorbance, or the like, mechanical means which measures a viscosity of blood plasma, or the like may be used. In the present specification presented below, the method of the present invention will be described using a coagulation reaction measurement based on a scattered light quantity as an example.

While a reaction start time point of a coagulation reaction can be typically defined as a time point where calcium ions (for example, a calcium chloride solution) are mixed with a subject specimen and the coagulation reaction is started, other timings may be defined as the reaction start time point. A period of time during which the measurement of the coagulation reaction is continued can be, for example, several ten seconds to around seven minutes from the time point at which the reagent is mixed with the subject specimen. While the measurement time may be an optionally-set fixed value, the measurement time may conclude at a time point where an end of the coagulation reaction of each subject specimen is detected. During the measurement time, a measurement (photometry in a case where detection is performed optically) of a progress of the coagulation reaction can be repetitively performed at predetermined intervals. For example, measurements may be performed at 0.1-second intervals. A temperature of the mixed liquid during the measurement corresponds to normal conditions such as 30°C or higher and 40°C or lower and, preferably, 35°C or higher and 39°C or lower. In addition, various conditions of measurement can be appropriately set according to the specimen, the reagent, the measurement means, and the like.

A series of operations in the coagulation reaction measurement described above can be performed using an automated analyzer. Examples of the automated analyzer include the Automated Coagulation Analyzer CP3000 (manufactured by SEKISUI MEDICAL CO., LTD.). Alternatively, a part of the operations may be performed manually. For example, the subject specimen can be prepared by hand and subsequent operations can be performed by an automated analyzer.

1.2. Acquisition of coagulation reaction curve

A coagulation reaction curve P(i) is acquired from the coagulation reaction measurement described above. In this case, "i" represents the number of measurement points or a time from start of the coagulation reaction (also simply referred to as time). For example, when the measurement (photometry) interval is 0.1 seconds, "i" is represented as time = 0.1 × number of measurement points. In other words, P(i) may be a function of the number of measurement points or a function of time. In the present specification described below, P(i) may be abbreviated as simply P. Generally, the coagulation reaction curve P is measured values of a coagulation reaction measurement to which denoising or smoothing or, when necessary, zero point adjustment or curve relativization for adjusting an initial value are applied by ordinary means. Figure 1 shows an example of a coagulation reaction curve. In Figure 1, an axis of abscissa represents time and an axis of ordinate represents a scattered light quantity. Since the coagulation reaction of the mixed liquid progresses as time elapses, the scattered light quantity increases.

When necessary, from the acquired coagulation reaction curve P, a differential curve such as a reaction rate curve (primary differentiation) or a reaction acceleration curve (secondary differentiation) may be acquired. Differential processing of a coagulation reaction curve can be performed by any method such as calculating an intra-interval average gradient value. In the present specification, a coagulation reaction curve, a primary differential curve such as a reaction rate curve, and a secondary differential curve such as a reaction acceleration curve will be also referred to as a "zero curve", a "primary curve", and a " secondary curve", respectively.

1.3. Detection of coagulation reaction end point Pe

In the method of the present invention, a coagulation reaction end point Pe in a coagulation reaction curve P of a subject specimen is detected. Pe can be determined according to any criterion such as a time point at which P reaches a plateau, a time point at which a primary curve of P decreases to 0 or a constant value after reaching a peak (refer to Japanese Patent Application No. 2020-068877), and an earliest point at which a ratio of an integrated value of P(i) in a small time slot falls below a threshold (for example, 1.001) (refer to International Application No. PCT/JP2020/048676 or examples to be described later). The detection of Pe may be performed after acquiring P until a predetermined measurement time or the detection of Pe may be performed in parallel with the acquisition of P and the acquisition of P may be ended once Pe is detected. For example, a procedure described in Japanese Patent Application No. 2020-068877 or International Application No. PCT/JP2020/048676 enables Pe to be detected in parallel with the acquisition of P (so-called real-time Pe detection).

As an embodiment of the method of the present invention, an example of a detection procedure of Pe based on the method described in International Application No. PCT/JP2020/048676 will be described in detail. A ratio of an integrated value of P(i) in a small time slot is defined as an integrated ratio Z(i) and calculated by the equation described below:

Z(i)={P(i+1)+P(i+2)+...+P(i+m)}/{P(i- m)+P(1-m+1)+...+P(i-1)}

In the equation above, i represents a measurement point number and m can be appropriately set based on a measurement condition, analysis items, and the like of a coagulation reaction and is, for example, m = 10 to 30. P(i) at an earliest measurement point or a time point at which Z(i) becomes smaller than a threshold Zs is detected as the coagulation reaction end point Pe. While Zs can be appropriately set according to analysis items, Zs is larger than 1 and equal to or smaller than 1.100 and, for example, in the case of an APTT measurement, Zs is preferably equal to or smaller than 1.050 and more preferably ranges from 1.010 to 1.001. In order to prevent an erroneous detection of Pe due to an early response anomaly or the like, the calculation of Z(i) is preferably performed after i reaches a predetermined calculation start point and P(i) equals or exceeds a predetermined value. In the present procedure, P(i) can be acquired, Z(i) can be calculated, and Pe can be detected in parallel while measuring a coagulation reaction.

1.4. Calculation of coagulation time

In the method of the present invention, a coagulation time of a subject specimen can be calculated based on a coagulation reaction curve P. The calculation of a coagulation time can be performed in accordance with any method. Examples of a method of calculating a coagulation time include: a method of calculating a time point where P(i) reaches N% of the coagulation reaction end point Pe as a coagulation time; a method of calculating a time point where a primary curve of P reaches N% of a maximum value thereof as a coagulation time; a method of adopting a point where a ratio of an integrated value of P(i) in a small time slot reaches a predetermined value as a calculation start point Te and calculating a time point where P(i) reaches N% of P(Te) as a coagulation time (refer to JP-A-H6-249855); a method of calculating a coagulation time based on a change over time of an integrated value of P(i) in a small time slot (refer to International Application No. PCT/JP2020/048676 or examples to be described later); a method of calculating a coagulation time based on a weighted average time of a primary curve of P (refer to Japanese Patent Application No. 2020-039344); and a method of adopting a point where a primary curve of P reaches a predetermined value after reaching a maximum value thereof as a calculation start point Te and calculating a time point where P(i) reaches N% of P(Te) as a coagulation time (refer to Japanese Patent Application No. 2020-068877).

2. Calculation of T(X)

Once Pe is detected, T(X) which represents a measurement point or a time at which the coagulation reaction curve P(i) reaches X% of Pe is calculated. More specifically, T(X) is the number of measurement points or a time until P reaches X% of Pe from a coagulation reaction start time (time 0). In other words, the following equation is satisfied with respect to T(X).

$$P(T(X)) = Pe \times X\%$$

In this case, X is a variable which is larger than 0 and equal to or smaller than 100. Therefore, T(X) is a function of the variable X. In the method of the present invention, X preferably ranges from 1 to 100 and more preferably ranges from 5 to 95. For example, each value of X may be a value separated by 1 or more (in other words, increment of $X \geq 1$) or a value separated by 5 or more (in other words, increment of $X \geq 5$). In an example, X is a variable which changes at an increment of 1 within a range from 1 to 100 (in other words, X = {1, 2, 3, ..., 97, 98, 99, 100}). In another example, X is a variable which changes at an increment of 5 within a range from 5 to 95 (in other words, X = {5, 10, 15, ..., 90, 95}).

Alternatively, T(X) may be represented as a mean from T(X - K) to T(X + K). In this case, K is preferably smaller than the increment of X described earlier. For example, when K = 2, T(X) can be represented as a mean from T(8) to T(12) when X = 10 or represented as a mean from T(13) to T(17) when X = 15.

Figure 2 is a diagram describing T(X). The diagram shows a coagulation reaction curve indicating points at which 10 to 90% of Pe are reached and time points where 10%, 50%, and 90% of Pe are reached are respectively denoted as T(10), T(50), and T(90). A time point tPe of Pe corresponds to T(100). Note that in the present specification and the drawings, T(X) may be notated as "TX" without the parentheses. Generally, as shown in Figure 7B to be described later, a T(X) curve which is obtained by plotting T(X) with respect to X rises abruptly when X is between 0 and near 5, the rise becomes gradual and approaches a linear shape when X is between 10 to near 15 and approximately 80, and once again rises abruptly when X is near 90.

3. Detection of anomaly in coagulation reaction based on shape of T(X) curve

In the method of the present invention, an anomaly in a coagulation reaction of a subject specimen is detected based on a shape of a T(X) curve obtained by plotting T(X) with respect to X (hereinafter, simply referred to as a shape of a T(X) curve). In a preferable embodiment, in the method of the present invention, an index R which reflects the shape of the T(X) curve of the subject specimen is calculated. An anomaly in a coagulation reaction of the subject specimen is detected based on a value of the index R.

3.1. Calculation of index R

The index R is a parameter capable of reflecting a shape of the T(X) curve. In an embodiment, two, three, or four or more values of T(X) are calculated in order to calculate R. In an embodiment, two values of X, $X_1$ and $X_2$, are set and two values of T(X), $T(X_1)$ and $T(X_2)$, are calculated. In another embodiment, three values of X, $X_1$, $X_2$, and $X_3$, are set and three values of T(X), $T(X_1)$, $T(X_2)$, and $T(X_3)$, are calculated. In yet another embodiment, four values of X, $X_1$, $X_2$, $X_3$, and $X_4$, are set and four values of T(X), $T(X_1)$, $T(X_2)$, $T(X_3)$, and $T(X_4)$, are calculated. In yet another embodiment, five values of X, $X_1$, $X_2$, $X_3$, $X_4$, and $X_5$, are set and five values of T(X), $T(X_1)$, $T(X_2)$, $T(X_3)$, $T(X_4)$, and $T(X_5)$, are calculated. In yet another embodiment, six values of X, $X_1$, $X_2$, $X_3$, $X_4$, $X_5$, and $X_6$, are set and six values of T(X), $T(X_1)$, $T(X_2)$, $T(X_3)$, $T(X_4)$, $T(X_5)$, and $T(X_6)$, are calculated. In yet another embodiment, eight values of X, $X_1$ to $X_8$, are set and eight values of T(X), $T(X_1)$ to $T(X_8)$, are calculated. In the present specification hereinafter, $X_k$ (for example, $X_1$ to $X_8$) in an equation which represents each index R is independently specified in each equation. However, as described above, since the T(X) curve can generally abruptly change in ranges of X of less than 5 and more than 90, the index R is preferably calculated from T(X) outside of such ranges. X is preferably 5 or more, more preferably 6 or more, even more preferably 7 or more, even more preferably 9 or more, even more preferably 10 or more, and even more preferably 11 or more. On the other hand, X is 100 or less, preferably 99 or less, more preferably 95 or less, even more preferably 94 or less, even more preferably 90 or less, and even more preferably 89 or less.

In an embodiment, R can be a relative value of T(X) with respect to a given X. Preferably, R can be a T(X) ratio or, in other words, a ratio between $T(X_1)$ and $T(X_2)$, $[T(X_1)/T(X_2)]$. In this case, $X_1$ and $X_2$ are preferably respectively selected from a range from 5 to 100, where $X_1 \neq X_2$ and either $X_1 < X_2$ or $X_1 > X_2$ may be satisfied. In an embodiment, $X_1 > X_2$ is satisfied and $X_1$ preferably ranges from 7 to 99 and $X_2$ preferably ranges from 6 to 95, and $X_1$ more preferably ranges from 11 to 99 and $X_2$ more preferably ranges from 10 to 90. In another embodiment, $X_1 < X_2$ is satisfied and $X_1$ preferably ranges from 6 to 94 and $X_2$ preferably ranges from 7 to 95, and $X_1$ more preferably ranges from 9 to 89 and $X_2$ more preferably ranges from 10 to 90. In a more preferable embodiment, $X_1$ ranges from 20 to 100 and $X_2$ ranges from 10 to 14.

In an embodiment, R can be a rate of change (rate of interval change) of T(X) with respect to a given range of X. Preferably, R can be represented as $[(T(X_1)-T(X_3))/T(X_2)]$. In an embodiment, $X_1 > X_2$ is satisfied, $X_1$ preferably ranges from 10 to 100, $X_2$ preferably ranges from 10 to 90 and more preferably ranges from 40 to 60, and $X_3$ preferably ranges from 5 to 95.

In an embodiment, R can be a coefficient of variation of T(X) with respect to a given range of X. Preferably, R can be a coefficient of variation of T(X), CV $[T(X_1):T(X_2)]$, within a range of X = $X_1$ to $X_2$. In this case, $X_1 < X_2$ is satisfied, $X_1$ preferably ranges from 10 to 86, $X_2$ preferably ranges from 14 to 90, and $(X_2 - X_1)$ preferably ranges from 4 to 80.

3.2. Detection of anomaly in coagulation reaction

The calculated index R can indicate different distributions between a specimen exhibiting an anomaly in a coagulation reaction and a normal specimen (for example, refer to Figure 12 to be described later). Therefore, the index R can be used as an index for detecting an anomaly in a coagulation reaction. In the method of the present invention, presence or absence of an anomaly in a coagulation reaction of a subject specimen can be detected based on the index R.

For example, while a coagulation reaction curve of a normal specimen has a sigmoidal shape (in a case of a measurement based on a scattered light quantity), a coagulation reaction curve of an abnormal specimen exhibits an abnormal shape such as having a premature rise due to an early response anomaly, without an abrupt rise but having a linearly rising shape, and without a portion which reaches a plateau. As a result, the shape of the T(X) curve of an abnormal specimen exhibits a trend which differs from that of a normal specimen. The index R desirably reflects such an anomaly in the shape of the T(X) curve.

In an embodiment, $R = [T(X_1)/T(X_2)]$, $X_1 > X_2$ is satisfied ($X_1$ preferably ranges from 7 to 99 and $X_2$ preferably ranges from 6 to 95, $X_1$ more preferably ranges from 11 to 99 and $X_2$ more preferably ranges from 10 to 90, and $X_1$ even more preferably ranges from 20 to 100 and $X_2$ even more preferably ranges from 10 to 14), and R of an abnormal specimen becomes larger than that of a normal specimen.

In another embodiment, $R = [T(X_1)/T(X_2)]$, $X_1 < X_2$ is satisfied ($X_1$ preferably ranges from 6 to 94 and $X_2$ preferably ranges from 7 to 95, and $X_1$ more preferably ranges from 9 to 89 and $X_2$ more preferably ranges from 10 to 90), and R of an abnormal specimen becomes smaller than that of a normal specimen.

In another embodiment, $R = [(T(X_3) - T(X_5))/T(X_4)]$ (where $X_3 > X_5$ is satisfied, $X_3$ preferably ranges from 10 to 100, $X_4$ preferably ranges from 10 to 90, $X_5$ preferably ranges from 5 to 95), and R of an abnormal specimen becomes larger than that of a normal specimen. In a preferable embodiment, $X_3$ ranges from 65 to 90, $X_4$ ranges from 40 to 60, and $X_5$ ranges from 7 to 13.

In another embodiment, $R = CV [T(X_6):T(X_7)]$ (where $X_6$ preferably ranges from 10 to 86, $X_7$ preferably ranges from 14 to 90, and when ($X_7 - X_6 = 4$ to 80), R of an abnormal specimen becomes larger than that of a normal specimen.

In an embodiment, presence or absence of an anomaly in a coagulation reaction of a subject specimen can be detected by comparing the index R with respect to the subject specimen to a reference value. The reference value can be determined prior to implementing the method of the present invention. The reference value can be determined based on the index R from a given specimen group.

In an example, the index R of each specimen is calculated from a specimen group including a specimen which exhibits an anomaly in a coagulation reaction (hereinafter, also simply referred to as an "abnormal specimen") and a specimen which does not exhibit an anomaly in a coagulation reaction (hereinafter, also simply referred to as a "normal specimen") and a reference value for detecting an anomaly in a coagulation reaction can be determined based on the calculated indexes R.

In another example, the index R of each specimen is calculated from a group of normal specimens and a reference value for detecting an anomaly in a coagulation reaction can be determined based on the calculated indexes R.

In another example, the index R of each specimen is calculated from a group of abnormal specimens and a reference value for detecting an anomaly in a coagulation reaction can be determined based on the calculated indexes R.

Therefore, in a preferable embodiment of the method of the present invention, a threshold Th for distinguishing an abnormal specimen and a normal specimen from each other can be set. As described earlier, the threshold Th can be determined based on the index R from a given specimen group. The threshold Th is determined so that R of an abnormal specimen is equal to or smaller than the threshold Th or equal to or larger than the threshold Th.

Since a shape of a reaction curve (in other words, a T(X) curve) of a normal specimen can differ per analysis item or according to a difference in Zs values, the threshold Th can be appropriately set for each analysis item or each Zs value. In addition, when there is a possibility that a difference in coagulation times may arise among lots of a reagent, the threshold Th can be set for each reagent lot.

For example, with an index R a value of which in an abnormal specimen is smaller than that in a normal specimen, the threshold Th can be set between a maximum value of R in abnormal specimens and a minimum value of R in normal specimen group. Alternatively, with an index R a value of which in an abnormal specimen is smaller than that in a normal specimen, the threshold Th can be set to Th = $[M - (K \times SD)]$ or smaller, where M and SD are, respectively, a mean and a standard deviation of the index R obtained from a group of normal specimens, and while K can be set to any number, for example, K ranges from 2 to 15, preferably ranges from 2 to 10, and more preferably ranges from 2 to 5. For example, K is preferably 2 or larger, more preferably 3 or larger, even more preferably 4 or larger, and even more preferably 5. When the index R of a subject specimen is equal to or smaller than the threshold Th, the subject specimen can be detected to have an anomaly in a coagulation reaction.

On the other hand, with an index R a value of which in an abnormal specimen is larger than that in a normal specimen, the threshold Th can be set between a minimum value of R in abnormal specimens and a maximum value of R in normal specimen group. Alternatively, with an index R a value of which in an abnormal specimen is larger than that in a normal specimen, the threshold Th can be set to Th = $[M + (K \times SD)]$ or larger, where M, K, and SD are defined in a similar manner to that described above. When the index R of a subject specimen is equal to or larger than the threshold Th, the subject specimen can be detected to have an anomaly in a coagulation reaction.

A plurality of indexes R can be used in combination in order to detect an anomaly in a coagulation reaction. For example, two or more selected from the group consisting of the T(X) ratio, the rate of change of T(X), and a coefficient of variation of T(X) described above can be used in combination. For example, a combination of a T(X) ratio and a rate of change of T(X), a combination of T(X) ratios with respect to different values of X, and a combination of rates of change of T(X) with respect to different values of X can be used. The threshold Th can be set for each of a plurality of indexes R. For example, thresholds Th1 and Th2 can be respectively set with respect to indexes R1 and R2. Alternatively, in order to detect an anomaly in a coagulation reaction, two or more thresholds Th can be set with respect to one index R. For example, thresholds Th1 and Th2 can be set with respect to an index R1.

3.3. Discrimination of anomaly type

Typically, types of abnormalities (anomaly types) of a blood coagulation reaction are classified into the following three types: (1) an anomaly with an early response anomaly and a reaction curve of which monotonously rises after a start of measurement (hereinafter, referred to as an "anomaly type 1", refer to specimen 18 in Example 2); (2) an anomaly with an early response anomaly and a reaction curve of which exhibits a biphasic rise (hereinafter, referred to as an "anomaly type 2", refer to specimen 19 in Example 2); and (3) an anomaly without an early response anomaly but a reaction curve of which linearly rises from an early stage of the beginning of the reaction (hereinafter, referred to as an "anomaly type 3", refer to specimen 20 in Example 2). Examples of the anomaly described in (3) include a reaction which linearly rises from an early stage and the measurement ends midway through the reaction (hereinafter, such a reaction will be referred to as a "linear end"). A value of the index R may differ depending on the anomaly type. A type of an anomaly can be discriminated using the index R. Hereinafter, a method of discriminating an anomaly type of an abnormal specimen by the method of the present invention will be described.

In an embodiment, two indexes R (R1 and R2) and two thresholds Th1 and Th2 are used. R1 is compared with Th1 and R2 is compared with Th2. The anomaly type of a subject specimen is discriminated based on whether or not R1 and R2 had reached the respective thresholds. For example, when neither R1 nor R2 with respect to a subject specimen has not reached the respective thresholds, the subject specimen can be discriminated to be normal, when both R1 and R2 has reached the respective thresholds, the subject specimen can be discriminated to be the anomaly type 1, when R1 has not reached Th1 but R2 has reached Th2, the subject specimen can be discriminated to be the anomaly type 2, and when R1 has reached Th1 but R2 has not reached Th2, the subject specimen can be discriminated to be the anomaly type 3.

In the present specification, "R with respect to a subject specimen reaches a threshold" means that R of the subject specimen is equal to or larger than the threshold when (R of normal specimen) < (R of abnormal specimen) and means that R of the subject specimen is equal to or smaller than the threshold when (R of normal specimen) > (R of abnormal specimen).

In an embodiment, first, normal specimens and abnormal specimens are separated from each other based on the threshold Th1 of R1. In other words, R1 is compared with Th1 and whether a subject specimen is normal or abnormal is discriminated based on whether or not R1 has reached Th1. Next, R2 is calculated with respect to an abnormal specimen. Two thresholds Th2 and Th3 (Th2 < Th3) are set with respect to R2. When R2 of the abnormal specimen has not reached Th2, the subject specimen is discriminated to be the anomaly type 2, when R2 has reached Th3, the subject specimen is discriminated to be the anomaly type 1, and when R2 has reached Th2 but not Th3, the subject specimen is discriminated to be the anomaly type 3.

The following may be used as the index R. The indexes R listed below may be used as R1 or as R2. Alternatively, different values of X may be applied to a same equation of the index R and respectively used as R1 and R2.

$$- \quad R \;=\; [T(X_1)/T(X_2)]$$

(where

X_1 > X_2 is satisfied and $X_1$ preferably ranges from 7 to 99 and $X_2$ preferably ranges from 6 to 95, $X_1$ more preferably ranges from 11 to 99 and $X_2$ more preferably ranges from 10 to 90, and $X_1$ even more preferably ranges from 20 to 100 and $X_2$ more preferably ranges from 10 to 14), or
$X_1 < X_2$ is satisfied and $X_1$ preferably ranges from 6 to 94 and $X_2$ preferably ranges from 7 to 95, and $X_1$ more preferably ranges from 9 to 89 and $X_2$ more preferably ranges from 10 to 90).

$$- \quad R \;=\; [(T(X_3) - T(X_5))/T(X_4)]$$

(where $X_3 > X_5$ is satisfied, $X_3$ preferably ranges from 10 to 100, $X_4$ preferably ranges from 10 to 90 and more preferably ranges from 40 to 60, and $X_5$ preferably ranges from 5 to 95).

$$- R = CV [T(X_6):T(X_7)]$$

(where $X_6$ preferably ranges from 10 to 86, $X_7$ preferably ranges from 14 to 90, and $(X_7 - X_6)$ preferably ranges from 4 to 80).

The threshold of each R can be determined in accordance with the procedure presented in 3.2 described above.

3.4. Detection example

An example of a detection procedure of an anomaly in a coagulation reaction using the index R will be described.

$$\text{Procedure A-1)} \quad R = T(X_1)/T(X_2)$$

With $R = T(X_1)/T(X_2)$ which is a relativization of a T(X) curve, R of an abnormal specimen becomes larger than that of a normal specimen when $X_1 > X_2$ is satisfied and R of an abnormal specimen becomes smaller than that of a normal specimen when $X_1 < X_2$ is satisfied. A mean of R of normal specimens + K $\times$ SD (K > 2) can be used as the threshold Th. For example, $X_1$ ranging from 20 to 100, $X_2$ ranging from 10 to 14, and R of normal specimens + 5SD can be used as the threshold Th. When R of the subject specimen is equal to Th or is larger than Th, an anomaly in a coagulation reaction is detected in the subject specimen but, if not, the subject specimen is detected as a normal specimen (Table 1-A). Alternatively, when $X_1 < X_2$, R of normal specimens - 5SD can be used as the threshold Th. When R of the subject specimen is equal to Th or is smaller than Th, an anomaly in a coagulation reaction is detected in the subject specimen but, if not, the subject specimen is detected as a normal specimen. With the present index R, any of the anomaly types 1 to 3 can be detected. Furthermore, by combining with procedures B) to D) below, an anomaly type can be discriminated.

$$\text{Procedure A-2)} \quad R = (T(X_1) - T(X_3))/T(X_2)$$

A coagulation reaction curve of an abnormal specimen characteristically assumes "a curved shape which is wide in a time axis direction" than a coagulation reaction curve of a normal specimen which approaches a sigmoid. A width of the reaction curve is reflected on $T(X_1) - T(X_3)$ ($X_1 > X_3$). A relativization thereof, $[(T(X_1)-T(X_3))/T(X_2)]$, is used as the index R. Preferably, $X_1$ ranges from 65 to 90, $X_2$ ranges from 40 to 60, and $X_3$ ranges from 7 to 13. A mean of R of normal specimens + K $\times$ SD (K > 2) can be used as the threshold Th. Preferably, R of normal specimens + 5SD can be used as Th. When R of the subject specimen is equal to or larger than Th or larger than Th, an anomaly in a coagulation reaction is detected in the subject specimen but when R of the subject specimen is smaller than Th or equal to or smaller than Th, the subject specimen is detected as a normal specimen (Table 1-A). With the present index R, any of the anomaly types 1 to 3 can be detected. Furthermore, by combining with procedures B) to D) below, an anomaly type can be discriminated.

R1=(T($X_1$)-T($X_3$))/T($X_2$) and R2=(T($X_4$)- T($X_6$))/T($X_5$).                                            Procedure B)

Two indexes, namely, R1 = $[(T(X_1) - T(X_3))/T(X_2)]$ and R2 = $[(T(X_4) - T(X_6))/T(X_5)]$ are used. Preferably, $X_1$ ranges from 99 to 100, $X_2$ ranges from 50 to 80, $X_3$ ranges from 5 to 12, $X_4$ ranges from 70 to 80, $X_5$ ranges from 40 to 60, and $X_6$ ranges from 20 to 30. As thresholds, a mean of R1 of normal specimens + K1 $\times$ SD (K1 > 2) can be used as the threshold Th1 of R1 and a mean of R2 of normal specimens + K2 $\times$ SD (K2 > 2) can be used as the threshold Th2 of R2. Preferably, R1 of normal specimens + 5SD can be used as Th1 and R2 of normal specimens + 5SD can be used as Th2. When R1 or R2 of the subject specimen is equal to or larger than each threshold or larger than the threshold, an anomaly in a coagulation reaction is detected in the subject specimen. Furthermore, an anomaly type is discriminated based on whether or not either R1 or R2 exceeds a threshold (Table 1-B). For example, when R1 of a subject specimen is larger than Th1 and R2 is larger than Th2, the subject specimen can be discriminated as the anomaly type 1, when R1 of the subject specimen is larger than Th1 but R2 is not larger than Th2, the subject specimen can be discriminated as the anomaly type 2, and when R1 of the subject specimen is not larger than Th1 but R2 is larger than Th2, the subject specimen can be discriminated as the anomaly type 3.

$$R1=(T(X_1)-T(X_3))/T(X_2), R2=CV\,[T(X_4):T(X_5)] \hspace{3cm} \text{Procedure C)}$$

The "reaction progressing stage" of a normal specimen group characteristically has small variability of a T(X) curve. In consideration thereof, an abnormal specimen can be detected using a coefficient of variation CV of T(X) of the reaction progressing stage as the index R. The coefficient of variation CV $[T(X_4):T(X_5)]$ is a value obtained by dividing a standard deviation of T(X) within a range represented as X = $X_4$ to $X_5$ by a mean. In the anomaly type 2 which exhibits a biphasic reaction, CV decreases at a time following an end of a first phase of the reaction.

Therefore, in the present procedure, first, R1 is used to detect an abnormal specimen in accordance with procedure A. Next, with respect to the detected abnormal specimen, R2 = CV $[T(X_4):T(X_5)]$ (where $X_4$ ranges from 20 to 66, $X_5$ ranges from 24 to 70, and $(X_5 - X_4)$ ranges from 4 to 80 and preferably from 10 to 80) is calculated. A mean of R of normal specimens + K × SD (K > 2) can be used as the threshold Th2 of R2. Preferably, R of normal specimens + 5SD can be used as Th2. When R2 of a subject specimen is Th2 or smaller than Th2, the subject specimen is detected as the anomaly type 2. Furthermore, a second threshold Th3 with respect to R2 may be used and a mean of R of normal specimens + K × SD (K > 9) can be used as the threshold Th3. Preferably, R of normal specimens + 15SD can be used as Th3. When R2 of a subject specimen is equal to or larger than Th3 or larger than Th3, the subject specimen is detected as the anomaly type 1 (Table 1-C). Furthermore, when R2 is between Th2 and Th3, a discrimination of the anomaly type 3 can be made. For example, an abnormal specimen is detected in accordance with procedure A (R1 = [(T(90) - T(10))/T(50)], Th1 = R of normal specimens + 5SD). Next, using R2 = CV [T(20):T(70)], Th2 = R of normal specimens + 5SD, and Th3 = R of normal specimens + 15SD, an abnormal specimen is differentiated as follows: when R2 is equal to or smaller than Th2, a discrimination of the anomaly type 2 can be made, when R2 is larger than Th2 and equal to or smaller than Th3, a discrimination of the anomaly type 3 can be made, and when R2 is larger than Th2, a discrimination of the anomaly type 1 can be made.

[Table 1]

Table 1-A

| Index | Th | |
|---|---|---|
| | R<Th | R≥Th |
| R | Normal | Abnormal |

Table 1-B

| | | R1 | |
|---|---|---|---|
| | | R1<Th1 | R1≥Th1 |
| R2 | R2<Th2 | Normal | Anomaly type 2 |
| | R2≥Th2 | Anomaly type 3 | Anomaly type 1 |

Table 1-C

| | | R2 | | |
|---|---|---|---|---|
| | | R2<Th2 | Th2≤R2<Th3 | R2≥Th3 |
| R1 | R1<Th1 | Normal | | |
| | R1≥Th1 | Anomaly type 2 | Anomaly type 3 | Anomaly type 1 |

A person skilled in the art will appreciate that as long as an anomaly type can be distinguished in discrimination criteria of an anomaly in a blood coagulation reaction described in the present specification, reference values of "smaller than a threshold" and "equal to or smaller than a threshold" are interchangeable and reference values of "larger than a threshold" and "equal to or larger than a threshold" are interchangeable.

3.5. Output of result

A detection result of an anomaly in a coagulation reaction can be output in any format. When necessary, information on a coagulation time of a subject specimen, accuracy of detection, or the like can be output together with presence or absence of an anomaly in a coagulation reaction of the subject specimen or a detection result of an anomaly type. Preferably, the presence or absence of an anomaly in a coagulation reaction of the subject specimen or a detection result of an anomaly type and a coagulation time are output together. The method of the present invention provides data indicating the presence or absence of an anomaly in a coagulation reaction. Data provided by the method of the present invention itself does not indicate a clinical diagnosis result.

3.6. Detection flow

As an embodiment, an exemplary flow for detecting an anomaly in a coagulation reaction by the method of the present invention is shown in Figure 3-A. Detailed procedures of the flow shown in Figure 3-A will be described below.

S00: Start measurement of coagulation reaction.
S01: Acquire coagulation reaction curve P(i) at time i.
S02: Determine presence or absence of detection of Pe.

When Pe is detected, advance to S03.
When Pe is not detected, return to S01 and continue to acquire P(i).

S03: Calculate APTT based on P(i) and Pe.
S04: Calculate T(X) based on P(i) and Pe.
S05: Calculate index R based on T(X).
S06: Compare index R with threshold Th for detection of anomaly in coagulation reaction.

When coagulation reaction is determined to be normal, advance to S10.
When anomaly in coagulation is determined, advance to S20.

S10: Output result indicating fact that coagulation reaction is normal together with APTT.
S20: Output result indicating fact that anomaly in coagulation reaction has been detected together with APTT.

In an embodiment of the method of the present invention, a detection of Pe (detection of Pe in so-called real time) is performed in parallel with an acquisition of P, which enables an anomaly in a blood coagulation reaction to be immediately detected upon obtaining coagulation reaction measurement data necessary for calculating a blood coagulation time. Therefore, since the present embodiment enables a blood coagulation time to be obtained in real time and simultaneously enables the presence or absence of an anomaly in a blood coagulation reaction to be detected, the present embodiment contributes to improving analysis efficiency in automated analyzers.
As an embodiment of such real-time detection, an exemplary flow for detecting an anomaly in a coagulation reaction by the method of the present invention when detection of Pe is performed using an integrated ratio Z(i) of P(i) and a threshold Zs based on the method described in International Application No. PCT/JP2020/048676 is shown in Figure 3-B. Detailed procedures of the flow shown in Figure 3-B will be described below.

S00: Set initial value of Zs.
S01: Start measurement of coagulation reaction.
S02: Acquire coagulation reaction curve P(i) at time i.
S03: Calculate Z(i) from P(i).
S04: Detect P(i) as Pe when Z(i) is smaller than threshold Zs.

When Pe is detected, advance to S05.
When Pe is not detected, return to S02 and continue to acquire P(i).

S05: Calculate APTT based on P(i) and Pe.
S06: Calculate T(X) based on P(i) and Pe.
S07: Calculate index R based on T(X).
S08: Confirm that Zs is initial value.

If Zs is initial value, advance to S09.

If Zs is not initial value, advance to S30.

S09: Compare index R with threshold Th for detection of anomaly in coagulation reaction.

When coagulation reaction is determined to be normal, advance to S20.

When anomaly in coagulation is determined, advance to S10.

S10: Discriminate type of coagulation anomaly (whether or not reaction is "linear end").

If type is "linear end", advance to S11.

If type is not "linear end", advance to S21.

S11: Reset Zs, and return to S02 and continue to acquire P(i).

S20: Output result indicating fact that coagulation reaction is normal together with APTT.

S21: Output result indicating fact that anomaly in coagulation reaction has been detected together with APTT.

S30: Compare index R with threshold Th for detection of anomaly in coagulation reaction.

S31: Output result indicating fact that coagulation reaction is normal together with APTT.

S32: Output result indicating fact that anomaly in coagulation reaction has been detected together with APTT.

4. Application to other coagulation reaction measurement methods

A method for detecting an anomaly in a coagulation reaction according to the present invention has been described above using a case of a coagulation reaction measurement based on a scattered light quantity as an example. However, a person skilled in the art should be able to apply the method based on a scattered light quantity described above to methods using other coagulation reaction measurement methods (for example, blood coagulation reaction measurement methods based on transmittance, absorbance, viscosity, or the like) and, therefore, such an application is included in a scope of the present invention.

5. Program and apparatus

The method for detecting an anomaly in a coagulation reaction according to the present invention described above can be automatically performed using a computer program. Therefore, an aspect of the present invention is a program for performing the method for detecting an anomaly in a coagulation reaction according to the present invention described above. In a preferable embodiment, the program according to the present invention is a program for implementing the flow shown in Figure 3-A or 3-B described above. In addition, a series of steps of the method of the present invention described above can be automatically performed by an automated analyzer. Therefore, an aspect of the present invention is an apparatus for performing the method for detecting an anomaly in a coagulation reaction according to the present invention described above.

An embodiment of the apparatus according to the present invention will be described below. The embodiment of the apparatus according to the present invention is an automated analyzer 1 such as that shown in Figure 4. The automated analyzer 1 includes a control unit 10, an operation unit 20, a measurement unit 30, and an output unit 40.

The control unit 10 controls operations of the automated analyzer 1 as a whole. The control unit 10 can be constituted of, for example, one or more computers. The control unit 10 includes a CPU, a memory, a storage, a communication interface (I/F), and the like and performs processing of a command from the operation unit 20, control of operations of the measurement unit 30, storage and data analysis of measured data received from the measurement unit 30, storage of an analysis result, control of output of measured data and/or the analysis result by the output unit 40, and the like. Furthermore, the control unit 10 may be connected to other devices such as an external medium and a host computer. In the control unit 10, a computer which controls operations of the measurement unit 30 may be or may not be the same as a computer which performs analysis of measured data.

The operation unit 20 acquires input from an operator and transmits obtained input information to the control unit 10. For example, the operation unit 20 includes a user interface (UI) such as a keyboard or a touch panel. The output unit 40 outputs, under control by the control unit 10, measured data of the measurement unit 30 and an analysis result based on the measured data such as a coagulation reaction curve P, a coagulation reaction end point Pe, a coagulation time, T(X), an index R, and a detection result of an anomaly in a coagulation reaction in a subject specimen. For example, the output unit 40 includes a display apparatus such as a display.

The measurement unit 30 executes a series of operations for a blood coagulation test and acquires measured data of a coagulation reaction of a sample including a blood specimen. The measurement unit 30 includes various equipment and analysis modules necessary for a blood coagulation test such as a specimen container for storing a blood specimen, a reagent container for storing a test reagent, a reaction container for a reaction between the specimen and the reagent, probes for dispensing the blood specimen and the reagent to the reaction container, a light source, a measuring instrument for measuring scattered light or transmitted light from the sample in the reaction container, a data processing circuit which sends data from the measuring instrument to the control unit 10, and a control circuit which receives an instruction of the control unit 10 and which controls operations of the measurement unit 30.

The control unit 10 performs an analysis of the presence or absence of an anomaly in a coagulation reaction of a subject specimen based on data measured by the measurement unit 30. The present analysis can include an acquisition of the coagulation reaction curve P, a calculation of a coagulation time, a detection of the coagulation reaction end point Pe, and calculations of T(X) and the index R described above. Furthermore, the control unit 10 may perform a detection of an anomaly in a coagulation reaction using the calculated R. Alternatively, the coagulation reaction curve P and Pe may be acquired by the control unit 10 based on measured data from the measurement unit 30 or may be acquired by another device such as the measurement unit 30 and sent to the control unit 10. The control unit 10 may save various parameters (for example, a calculation formula for calculating R) to be used to calculate the coagulation time of a subject specimen, T(X), and R and to detect Pe. In addition, the control unit 10 may save a reference value (for example, the threshold Th described above) to be used to detect an anomaly in a coagulation reaction. Alternatively, the control unit 10 may import, when performing an analysis, the parameters or reference values saved in an external device or on a network.

The present analysis described above can be implemented by a program for performing the method of the present invention. Therefore, the control unit 10 can include a program for performing the method for detecting an anomaly in a coagulation reaction according to the present invention.

An analysis result obtained by the control unit 10 is sent to the output unit 40 to be output. The output can take any form such as a display on a screen, a transmission to a host computer, a printout, or the like. Output information from the output unit can include data of a coagulation reaction curve, the coagulation reaction end point Pe, a coagulation time, T(X), the index R, and a detection result of an anomaly in a coagulation reaction. Types of output information from the output unit can be controlled by the program according to the present invention.

Examples

While the present invention will be described below in greater detail by citing examples, the present invention is not limited to the following examples. Coagulation reaction measurements in the following examples are performed in accordance with the procedures of APTT measurement.

Method

1) Coagulation reaction measurement

As a reagent for measurement, Coagpia APTT-N (manufactured by SEKISUI MEDICAL CO., LTD.) which is a reagent for APTT measurement was used as a first reagent and Coagpia APTT-N Calcium Chloride Solution (25-mM calcium chloride solution manufactured by SEKISUI MEDICAL CO., LTD.) was used as a second reagent. A coagulation reaction measurement of samples including a specimen was performed using an Automated Coagulation Analyzer CP3000 (manufactured by SEKISUI MEDICAL CO., LTD.). After heating 50 $\mu$L of a specimen in a cuvette at 37°C for 45 seconds, 50 $\mu$L of the first reagent at approximately 37°C was added and, after a lapse of 171 seconds, 50 $\mu$L of the second reagent was added to start a coagulation reaction. The reaction was performed at 37°C. In the measurement of the coagulation reaction, the cuvette was irradiated with light with a wavelength of 660 nm from an LED light source, and a scattered light quantity of light scattered 90 degrees to the side was measured at 0.1-second intervals. The measurement time was set to 360 seconds.

2) Acquisition of coagulation reaction curve

After performing smoothing including denoising with respect to photometry data from each specimen, zero point adjustment was performed so that a scattered light quantity at a time point of start of photometry equaled zero to create a coagulation reaction curve P(i).

3) Detection of coagulation reaction end point Pe and determination of APTT

P(i) at an earliest time point at which an integrated ratio Z(i) of the coagulation reaction curve P(i) (refer to PCT/JP2020/048676) becomes smaller than an integrated ratio threshold Zs is detected as the coagulation reaction end point Pe. 1.001 or 1.010 was used as the integrated ratio threshold Zs. A time point T(50) at which P(i) reached 50% of Pe was calculated and determined as APTT. The integrated ratio Z(i) at the time point i was calculated as follows:

$$\text{Integrated ratio } Z(i) = Pb(i)/Pa(i)$$

$$Pa(i) = \text{sum from } P(i - 20) \text{ to } P(i - 1)$$

$$Pb(i) = \text{sum from } P(i + 1) \text{ to } P(i + 20)$$

Example 1

1) Specimen

A coagulation reaction measurement of a blood specimen (citric acid-added plasma) of a human was performed in accordance with methods 1) to 2) described earlier and a coagulation reaction curve P was acquired. A normal coagulation reaction curve P has a sigmoidal shape which includes three intervals of: a "reaction initial stage" in which the curve starts to gradually rise; a "reaction progressing stage" in which the curve abruptly rises and which includes an inflection point; and a "reaction later stage" in which the rise of the curve moderates and plateaus (becomes planarized). Among specimens the reaction curve P of which is normal (sigmoidal shape), 17 specimens indicating respectively different APTTs (specimens T(50) of which at Zs = 1.001 differ by approximately 10-second intervals from 20-plus seconds to 180-plus seconds) were selected and adopted as a normal specimen group (specimens 1 to 17).

2) Detection of Pe

With respect to the normal specimen group, coagulation reaction end points Pe when Zs = 1.001 and Zs = 1.010 are set were measured in accordance with the method 3) described earlier. Reaction curves P up to the detected Pe are respectively shown in Figures 5A and 5B. An axis of abscissa of the diagrams represents a reaction time from zero being a time point of addition of the second reagent (calcium chloride solution), and an axis of ordinate represents P which is represented by a scattered light quantity (scattered light quantity subjected to zero point adjustment). P may also be represented by a scattered light quantity in subsequent drawings.

3) Calculation of APTT

Figure 6A shows a time tPe at which P with respect to the normal specimen group (specimens 1 to 17) reaches Pe, and plots in the diagram represent tPe (PmaxT: O) when a maximum value Pmax of the reaction curve P is adopted as Pe and tPe (respectively, Zs = 1.001: + and Zs = 1.010: ×) with respect to Pe detected in accordance with the method 3) described earlier when Zs = 1.001 and Zs = 1.010 are set. Figure 6B shows APTTs with respect to the specimens 1 to 17, and plots in the diagram represent a time point T(50) when P reaches 50% of each Pe shown in Figure 6A.
As is apparent from Figures 6A and 6B, there was hardly any difference in calculated APTTs among the three types of Pe (PmaxT, Zs = 1.001, and Zs = 1.010 shown in Figure 6A). Differences of APTTs due to Zs = 1.001 and Zs = 1.010 with respect to APTT due to PmaxT were, respectively, 0.5% and 2.8% at maximum.

4) Calculation of T(X)

With respect to the 17 specimens, a time T(X) required by the reaction curve P to reach each X% of Pe detected in 2) was calculated based on the equation presented below. 100 values of X were set at increments of 1 from 1 to 100 to calculate T(1) to T(100). Note that T(50) when X = 50 corresponds to APTT.

$$P(T(X)) = Pe \times X\%$$

Figures 7A and 8A represent reaction curves P (respectively the same as Figures 5A and 5B) up to Pe when Zs = 1.001 (Figure 7A) and Zs = 1.010 (Figure 8A), and Figures 7B and 8B represent T(X) curves based on Figures 7A and 8A. Positions of the T(X) curves of the 17 specimens on the axis of ordinate differed depending on rising times of P or APTT. All of the T(X) curves in the normal specimens rose abruptly when X was between 0 and near 5, the rises became gradual and approached a linear shape when X was between 10 to near 15 and approximately 80, and once again rose abruptly when X was near 90.

T(X) ratios were calculated with respect to the 17 specimens. Figures 7C and 8C respectively show ratios of T(X) shown in Figures 7B and 8B, [T(X)/T(50)] (X = 1 to 100). Figures 7D and 8D are diagrams obtained by expanding Figures 7C and 8C in a direction of the axis of ordinate and a dashed-dotted line and a dashed-two dotted line in the diagrams respectively represent a mean of T(X)/T(50) + 3SD and a mean of T(X)/T(50) - 3SD (SD means standard deviation) with respect to the 17 specimens. T(X)/T(50) of the 17 specimens tended to be comparatively close to each other in the interval where X ranges from 5 to 50 but were more varied in intervals where X is smaller than 5 and larger than 50.

Example 2

1) Specimen

Three blood specimens (specimens 18, 19, and 20) with an abnormal reaction curve P were prepared. Specimens 18 and 19 were citric acid-added plasma of a human. With respect to the specimen 18, a reaction (early response anomaly) was observed from immediately after the start of measurement and the reaction monotonously rose as-is (refer to Figure 9A). With respect to the specimen 19, an early response anomaly was observed and a reaction rose in a biphasic manner (refer to Figure 9B). With respect to the specimen 20 which was a factor VIII-deficient plasma (Factor VIII Deficient; George King Bio-Medical, Inc., with an FVIII activity value of less than 1%), a reaction started at a time point where a certain period of time had elapsed from immediately after the start of measurement (in other words, no early response anomaly), the reaction linearly rose in an early state and subsequently gradually planarized but the reaction curve did not reach a plateau (refer to Figure 9C).

2) Coagulation reaction measurement

Reaction curves P of the abnormal specimens (specimens 18 to 20) were acquired and coagulation reaction end points Pe were detected in accordance with the methods 1) to 3) described earlier. T(X) (X = 1 to 100, increments of 1) was calculated in a similar manner to Example 1.
The reaction curves P up to Pe (when Zs = 1.001) of the specimens 18 to 20 are respectively shown in Figures 9A to 9C together with data of the normal specimen group. In addition, the reaction curves P up to Pe (when Zs = 1.010) of the specimens 18 to 20 are respectively shown in Figures 10A to 10C together with data of the normal specimen group. In the diagrams, data of the specimens 18 to 20 are depicted by dotted lines.
As shown in Figures 10A to 10C, Pe when Zs = 1.010 occurs earlier compared to Zs = 1.001. In addition, while APTTs (T(50)) of the specimens 18 to 20 when Zs = 1.001 were 52 seconds, 53 seconds, and 112 seconds, respectively, APTTs (T(50)) of the specimens 18 to 20 when Zs = 1.010 were 36 seconds, 52 seconds, and 103 seconds, respectively, which were shorter as compared to when Zs = 1.001. In some abnormal specimens, APTT was affected by Zs unlike in the normal specimen group described in Example 1. This suggests that a shape of a reaction curve of the abnormal specimens differs from that of normal specimens and, conceivably, the reaction curve does not exhibit a clear reaction initial stage, a clear reaction progressing stage, and a clear reaction later stage.

3) Detection of anomaly in reaction -1

[T(X)/T(50)] of the abnormal specimens was calculated in a similar manner to Example 1. [T(X)/T(50)] when Zs = 1.001 of the specimens 18 to 20 are respectively shown in Figures 9D to 9F together with data of the normal specimen group. In addition, [T(X)/T(50)] when Zs = 1.010 of the specimens 18 to 20 are respectively shown in Figures 10D to 10F together with data of the normal specimen group. In the diagrams, data of the specimens 18 to 20 is denoted by a dotted line, and a dashed-dotted line and a dashed-two dotted line respectively represent a mean of [T(X)/T(50)] + 3SD and a mean of [T(X)/T(50)] - 3SD with respect to the 17 normal specimens.
T(X)/T(50) of the specimens 18 and 20 was outside of the range of a mean of the normal specimen group $\pm$ 3SD (Figures 9D, 9F, 10D, and 10F). In addition, T(X)/T(50) of the specimen 19 was outside of the range of a mean of the normal specimen group $\pm$ 3SD when X was smaller than near 15% (Figures 9E and 10E). Therefore, it was shown that an abnormal specimen can be detected using $T(X_1)/T(X_2)$ as an index. Note that it is obvious that the denominator $T(X_2)$ of the index need not be T(50). For example, the denominator may be T(40) or T(60) as shown in Figure 11. In addition, as shown in Figures 9 to 11, $T(X_1)/T(X_2)$ of the abnormal specimens is larger than a mean with respect to the normal specimen group + 3SD when $X_1 > X_2$ and smaller than a mean with respect to the normal specimen group - 3SD when $X_1 < X_2$.
From the results described above, it is shown that the T(X) ratio [$T(X_1)/T(X_2)$] can be used as an index for detecting an anomaly in a coagulation reaction and that a mean with respect to the normal specimen group $\pm$ 3SD of the index can be used as thresholds for detecting the anomaly.

4) Detection of anomaly in reaction -2

A rate of interval change of T(X), $[(T(X_1) - T(X_3))/T(X_2)]$, with respect to the normal specimen group with 17 specimens of Example 1 and the three abnormal specimens was obtained. In Figure 12, the rate of interval change of T(X), $[(T(90) - T(10))/T(50)]$, is plotted with respect to APTT, with an upper half showing the plot when Zs = 1.001 and a lower half showing the plot when Zs = 1.010. In each diagram, a normal specimen is denoted by ×, abnormal specimens are denoted by ● (specimen 18), ▲ (specimen 19), and ■ (specimen 20), and a dotted line represents a mean of values of the 17 specimens of the normal specimen group ± 3SD which was adopted as a threshold for detecting an anomaly in a coagulation reaction.

In Figure 12, all of the three abnormal specimens (specimens 18 to 20) exceeded the threshold (the dotted line in the diagrams). It is shown that an anomaly in a coagulation reaction can be detected using the rate of interval change of T(X).

The index $[(T(90) - T(10))/T(50)]$ shown in Figure 12 can be represented as $[(T(90)/T(50)) - (T(10)/T(50))]$. As shown in Figure 13, the expression is represented by a difference W between a value of $(T(X)/T(50))$ when X = 90 and a value of $(T(X)/T(50))$ when X = 10 on a $T(X)/T(50)$ curve. Note that Figure 13 is a diagram obtained by changing a scale of the axis of ordinate of Figure 9F. As shown in Figure 13, with a normal specimen the reaction curve P of which has a sigmoidal shape, since $T(X)/T(50)$ hardly changes in the reaction progressing stage in which P abruptly rises, W assumes a small value. On the other hand, W assumes a larger value in abnormal specimens the reaction curve P of which does not take a sigmoidal shape.

From the results described above, it is shown that the rate of interval change of T(X), $[(T(X_1) - T(X_3))/T(X_2)]$, can be used as an index for detecting an anomaly in a coagulation reaction. Note that it is obvious that the denominator $T(X_2)$ of the index need not be T(50) as already shown in Figure 11.

5) Optimization of index

As an index, R(X) = T(X)/T(Y) (X = 1 to 100, Y = 10, 40, 50, 60, or 90) was used. A mean M(X) and a standard deviation SD(X) of R(X) with respect to the normal specimen group (specimens 1 to 17) and R(X) from the three abnormal specimens (specimens 18 to 20) were obtained and SDI(X) = $[(R(X) - M(X))/SD(X)]$ was calculated.

Figures 14A to 14E show SDI(X) of the three abnormal specimens (specimens 18 to 20). In each drawing, a solid line represents the specimen 18, a dotted line represents the specimen 19, and a dashed line represents the specimen 20. The SDIs of the abnormal specimens had a tendency of assuming a negative value when X < Y, a positive value when X > Y, and zero when X = Y, and approaching zero near X = 1 and near X = 100.

While an absolute value of the SDIs tended to decrease as Y increases, a maximum value of the absolute value of the SDIs exceeded 5. This indicates that, with respect to a mean of the normal specimen group, the index R(X) of the abnormal specimens may significantly deviate from 5SD at maximum. On the other hand, SDIs varied significantly among the specimens when Y = 10 and Y = 90. Therefore, it is shown that, when using the T(X) ratio $[T(X_1)/T(X_2)]$ or the rate of interval change of T(X), $[(T(X_1) - T(X_3))/T(X_2)]$, as an index for detecting an anomaly in a coagulation reaction, the denominator $X_2$ need only range from 10 to 90 and preferably range from 40 to 60. On the other hand, $X_1$ and $X_3$ may be set according to $X_2$ so that the absolute value of SDI increases.

With a specimen a reaction curve of which exhibits a biphasic property due to an initial reaction such as the specimen 19, the biphasic property may only appear in an interval in which a portion where SDI(X) differs from normal (for example, a portion in which an absolute value of SDI(X) is 5 or larger) is smaller than X corresponding to a coagulation time (for example, an interval in which X < 50 when APTT is T(50)). In such a specimen, an anomaly in a coagulation reaction can be considered to have no effect on the calculation of a coagulation time. Therefore, when the object is to calculate a coagulation time, a biphasic curve such as the specimen 19 need not necessarily be determined as an abnormal curve.

Example 3

1) Coagulation reaction measurement

988 specimens (human citric acid-added plasma, including both normal and abnormal specimens) APTT of which ranges from 23 seconds to 267 seconds were adopted as a control specimen group. A reaction curve P and a coagulation reaction end point Pe (Zs = 1.001 or Zs = 1.010) of the control specimen group were acquired in accordance with the methods 1) to 3) described earlier and T(X) (X = 1 to 100, increments of 1) was calculated in a similar manner to Example 1.

2) Detection of anomaly in coagulation reaction by index R

$$2-1) \quad [T(X_1)/T(X_2)]$$

The index R = [T(X$_1$)/T(X$_2$)] was obtained with respect to three abnormal specimens (specimens 18 to 20) and the control specimen group. Figures 15A to 15D represent plots of [T(X$_1$)/T(X$_2$)] (where X$_1$ ranges from 20 to 100 and X$_2$ ranges from 10 to 14) with respect to APTT when Zs = 1.010. In each diagram, the control specimen group is denoted by a + sign, abnormal specimens are denoted by ● (specimen 18), ▲ (specimen 19), and ■ (specimen 20), and a dotted line represents a mean of values of the control specimen group + 5SD as a threshold. R of all of the three abnormal specimens (specimens 18 to 20) exceeded the threshold.

$$2-2) \quad [(T(X_1) - T(X_3))/T(X_2)]$$

Next, the index R = [(T(X$_1$) - T(X$_3$))/T(X$_2$)] was obtained with respect to the three abnormal specimens (specimens 18 to 20) and the control specimen group. Figures 16A to 16H represent plots of [(T(X$_1$) - T(X$_3$))/T(X$_2$)] (where X$_1$ ranges from 65 to 90, X$_2$ ranges from 40 to 60, and X$_3$ ranges from 7 to 13) with respect to APTT when Zs = 1.010. In each diagram, signs of the control specimen group and the abnormal specimens are the same as in Figure 15 and a dotted line represents a mean of values of the control specimen group + 5SD. R of all of the three abnormal specimens (specimens 18 to 20) exceeded the threshold.

$$2-3) \quad CV \ [T(X_1):T(X_2)]$$

The index R = CV [T(X$_1$):T(X$_2$)] was obtained with respect to the three abnormal specimens (specimens 18 to 20) of Example 2 and the control specimen group of Example 3. Figures 17A to 17C represent plots of CV [T(X$_1$):T(X$_2$)] (where X$_1$ is 10 and X$_2$ ranges from 14 to 90) with respect to APTT when Zs = 1.010. In each diagram, signs of the control specimen group and the abnormal specimens are the same as in Figure 15 and a dotted line represents a mean of values of the control specimen group + 5SD as a threshold. R of all of the three abnormal specimens (specimens 18 to 20) exceeded the threshold.

Comparative example 1

[T(X$_1$) - T(X$_2$)] was obtained as the index R with respect to the abnormal specimens (specimens 18 to 20) and the control specimen group (Zs = 1.001). Figure 18 represents a plot of [T(X$_1$) - T(X$_2$)] with respect to APTT, where X$_1$ = 50 + a and X$_2$ = 50 - a, and a is set to 5, 10, and 40 in an order of Figures 18A to 18C. In each diagram, signs of the control specimen group and the abnormal specimens are the same as in Figure 15 and a dotted line represents a mean of values of the control specimen group + 5SD which is adopted as a threshold. Since the index R increases depending on APTT and, therefore, a specimen with a delayed APTT may exceed the threshold not only among abnormal specimens but also among the control specimen group, detecting an abnormal specimen with the index R is conceivably difficult.

Example 4

Representative types of abnormalities (anomaly types) of a reaction include: the anomaly with an early response anomaly and a reaction curve of which monotonously rises after start of measurement such as in the specimen 18 (hereinafter, referred to as the "anomaly type 1"); the anomaly with an early response anomaly and a reaction curve of which exhibits a biphasic rise such as in the specimen 19 (hereinafter, referred to as the "anomaly type 2"; and the anomaly without an early response anomaly but a reaction curve of which linearly rises from an early stage of the beginning of the reaction such as in the specimen 20 (hereinafter, referred to as the "anomaly type 3"). A possibility of implementation of discrimination of an anomaly type in accordance with the procedures B to C (Tables 1-B to 1-C) described earlier was verified using the specimens of Example 3.

1) Discrimination of anomaly type in accordance with procedure B

Results shown in Figures 19 to 20 suggest a possibility that anomaly types 1 to 3 can be discriminated using the procedure B (R1 = $(T(X_1) - T(X_3))/T(X_2)$ and R2 = $(T(X_4) - T(X_6))/T(X_5)$). For example, R1 = $(T(X_1) - T(X_3))/T(X_2)$ (where $X_1$ ranges from 99 to 100, $X_2$ ranges from 50 to 80, and $X_3$ ranges from 5 to 12) and R2 = $(T(X_4) - T(X_3))/T(X_3)$ (where $X_4$ ranges from 70 to 80, $X_5$ ranges from 40 to 60, and $X_3$ ranges from 20 to 30) are set. The anomaly type 3 can be discriminated when R1 is smaller than the threshold and R2 is equal to or larger than the threshold, the anomaly type 2 can be discriminated when R2 is smaller than the threshold and R1 is equal to or larger than the threshold, and the anomaly type 1 can be discriminated when both R1 and R2 are equal to or larger than the threshold. The specimen 20 belonging to the anomaly type 3 which is detected in the present procedure is factor VIII-deficient plasma. Conventionally, a coagulation factor deficiency was generally discriminated by a cross-mixing test of a specimen with a prolonged coagulation time. In contrast, in the present invention, a coagulation time of a subject specimen can be measured and an anomaly in coagulation ability attributable to a coagulation factor deficiency can be discriminated by simpler procedures. Note that while a part of the control specimens exceeded the threshold in Figure 19, it was confirmed that the coagulation reaction curves of the specimens tend to have shapes similar to that of the specimen 19 (anomaly type 2) .

2) Discrimination of anomaly type in accordance with procedure C

As shown in Figure 9, a change in the reaction curve P of a normal specimen group is gradual in the reaction initial stage and the reaction later stage but abrupt in the reaction progressing stage. This characteristic is represented by d(X) = $[T(X) - T(X - 1)]$ as a variation of T(X) as shown in Figures 21A to 21C. In other words, while d(X) in the reaction progressing stage (X = approximately 15 to 80) decreases in a normal specimen but partially or entirely increases according to a shape of an anomaly in an abnormal specimen.

Figure 22 respectively shows, with respect to the three abnormal specimens (specimens 18 to 20) and the control specimen group of Example 3, a plot of CV $[T(X_1):T(X_2)]$ (where $X_1$ ranges from 20 to 66, $X_2$ ranges from 24 to 70, and $(X_2 - X_1)$ ranges from 4 to 80) with respect to APTT. In each diagram, signs of the control specimen group and the abnormal specimens are the same as in Figure 15 and a dotted line represents a mean of values of the control specimen group + 5SD which is adopted as a threshold for detecting an anomaly in a reaction. All of the CVs shown in Figure 22 declined in an order of the specimen 18 (anomaly type 1), the specimen 20 (anomaly type 3), and the specimen 19 (anomaly type 2). Therefore, it is shown that, for example, by detecting abnormal specimens from normal specimens with R1 = $(T(90) - T(10))/T(50)$ and subsequently classifying the abnormal specimens with R2 = CV $[T(20):T(70)]$, the anomaly types 1 to 3 can be discriminated.

## Claims

1. A method for detecting an anomaly in a blood coagulation reaction, comprising:

    1) detecting a coagulation reaction end point Pe in a coagulation reaction curve of a subject blood specimen;
    2) calculating T(X), wherein T(X) represents a measurement point or time at which the coagulation reaction curve reaches X% of Pe and X denotes a variable larger than 0 and equal to or smaller than 100; and
    3) detecting the anomaly in the blood coagulation reaction of the subject blood specimen based on T(X).

2. The method according to claim 1, wherein 3) comprises calculating an index R based on T(X) and detecting presence or absence of the anomaly in the blood coagulation reaction of the subject blood specimen based on the index R.

3. The method according to claim 2, wherein the index R is at least one selected from the group consisting of a ratio of T(X) in a given range of X, a rate of change of T(X) in a given range of X, and a coefficient of variation of T(X) in a given range of X.

4. The method according to claim 3, wherein:

    the ratio of T(X) in the given range of X is $[T(X_1)/T(X_2)]$;
    the rate of change of T(X) in the given range of X is $[(T(X_3) - T(X_5))/T(X_4)]$; and
    the coefficient of variation of T(X) in the given range of X is a coefficient of variation of T(X) in a range from $X_6$ to $X_7$.

5. The method according to any one of claims 2 to 4, comprising detecting presence or absence of the anomaly in the blood coagulation reaction of the subject blood specimen by comparing the index R with a given threshold.

6. The method according to claim 5, wherein the threshold is determined based on the index R acquired from a group of specimens.

7. The method according to claim 5, wherein the anomaly in the blood coagulation reaction of the subject blood specimen is detected when:

   the threshold is equal to or larger than "mean + 2 × standard deviation" of index R acquired from a group of normal specimens, and the index R of the subject blood specimen is equal to or larger than the threshold, or the threshold is equal to or smaller than "mean - 2 × standard deviation" of index R acquired from a group of normal specimens, and the index R of the subject blood specimen is equal to or smaller than the threshold.

8. The method according to claim 1, wherein 3) comprises calculating indexes R1 and R2 based on T(X) and detecting a type of the anomaly in the blood coagulation reaction of the subject blood specimen based on the indexes R1 and R2.

9. The method according to claim 8, wherein

   the index R1 is a rate of change of T(X) in a first given range of X, and
   the index R2 is
   a rate of change of T(X) in a second given range of X or a coefficient of variation of T(X) in a given range of X.

10. The method according to claim 9, wherein

    the rate of change of T(X) in the first given range of X is $[(T(X_1) - T(X_3))/T(X_2)]$ ;
    the rate of change of T(X) in the second given range of X is $[(T(X_4) - T(X_6))/T(X_5)]$ ; and
    the coefficient of variation of T(X) in the given range of X is a coefficient of variation of T(X) in a range from $X_7$ to $X_8$.

11. The method according to claim 9 or 10, comprising comparing R1 with a threshold Th1 and comparing R2 with a threshold Th2, and

    detecting the type of the anomaly in the blood coagulation reaction of the subject blood specimen based on whether or not R1 and R2 had reached the respective thresholds, wherein
    R1 is the rate of change of T(X) in the first given range of X and R2 is the rate of change of T(X) in the second given range of X.

12. The method according to claim 9 or 10, comprising

    comparing R1 with a threshold Th1 and, when R1 had reached Th1, detecting that the subject blood specimen has the anomaly in the blood coagulation reaction,
    comparing R2 of the subject blood specimen having been detected to have the anomaly in the blood coagulation reaction with thresholds Th2 and Th3, and
    detecting the type of the anomaly in the blood coagulation reaction of the subject blood specimen based on whether or not R2 had respectively reached Th2 and Th3, wherein
    R1 is the rate of change of T(X) in the first given range of X and R2 is the coefficient of variation of T(X) in the given range of X.

13. The method according to claim 11, wherein the threshold Th1 is determined based on the index R1 acquired from a group of specimens and the threshold Th2 is determined based on the index R2 acquired from the group of specimens.

14. The method according to claim 12, wherein the threshold Th1 is determined based on the index R1 acquired from a group of specimens and the thresholds Th2 and Th3 are determined based on the index R2 acquired from the group of specimens.

15. The method according to claim 1, further comprising calculating a coagulation time of the subject blood specimen.

16. The method according to claim 1, wherein the detection of Pe is performed in parallel to acquisition of the coagulation reaction curve of the subject blood specimen, and the acquisition of the coagulation reaction curve is ended once Pe is detected.

**17.** A program for implementing the method according to claim 1.

**18.** An apparatus for implementing the method according to claim 1.

Fig. 1

Fig. 2

# Fig. 3-A

```
┌─────────────────────────┐
│  START MEASUREMENT OF   │  S00
│  COAGULATION REACTION   │
└─────────────────────────┘
              │
              │
┌─────────────────────────┐
│  MEASURE COAGULATION    │  S01
│  REACTION ACQUIRE P(i)  │
└─────────────────────────┘
              │
                              S02
        ◇ Pe DETECTED? ◇
                              No
             │
            Yes
┌─────────────────────────┐
│ CALCULATE APTT BASED ON P(i) │  S03
└─────────────────────────┘
              │
┌─────────────────────────┐
│ CALCULATE T(X) BASED ON P(i) │  S04
└─────────────────────────┘
              │
┌─────────────────────────┐
│ CALCULATE INDEX R BASED ON T(X) │  S05
└─────────────────────────┘
              │
                              S06
     ◇ COMPARE INDEX      ◇   DETERMINE AS
         R WITH Th            ABNORMAL
             │
         DETERMINE AS
         NORMAL
┌─────────────────────┐       ┌──────────────────┐
│ OUTPUT NORMAL RESULT │  S10  │ OUTPUT ABNORMAL  │  S20
└─────────────────────┘       │     RESULT       │
                              └──────────────────┘
```

25

# Fig. 3-B

| SET INITIAL VALUE TO Zs | S00 |

| START MEASUREMENT OF COAGULATION REACTION | S01 |

| MEASURE COAGULATION REACTION ACQUIRE P(i) | S02 |

| CALCULATE Z(i) FROM P(i) | S03 |

S04

Pe DETECTED? — No

Yes

| CALCULATE APTT BASED ON P(i) | S05 |

| CALCULATE T(X) BASED ON P(i) | S06 |

| CALCULATE INDEX R BASED ON T(X) | S07 |

S08

IS Zs INITIAL VALUE? — No (RESET VALUE)

Yes (INITIAL VALUE)

COMPARE INDEX R WITH Th — S09 — DETERMINE AS NORMAL

DETERMINE AS ABNORMAL

| OUTPUT NORMAL RESULT | S20 |

DISCRIMINATE ANOMALY TYPE — S10 — OTHER THAN LINEAR END

LINEAR END

| OUTPUT ABNORMAL RESULT | S21 |

| RESET Zs | S11 |

COMPARE INDEX R WITH Th — S30 — DETERMINE AS ABNORMAL

DETERMINE AS NORMAL

| OUTPUT NORMAL RESULT | S31 | | OUTPUT ABNORMAL RESULT | S32 |

Fig. 4

**MEASUREMENT UNIT** (30)

CONTROL CIRCUIT

SPECIMEN CONTAINER → SPECIMEN PROBE

REAGENT CONTAINER → REAGENT PROBE

LIGHT SOURCE → REACTION CONTAINER → DETECTOR → DATA PROCESSING CIRCUIT

**CONTROL UNIT** (10)

CPU

MEMORY

STORAGE

COMMUNICATION I/F

**OPERATION UNIT** (20)

UI

**OUTPUT UNIT** (40)

DISPLAY APPARATUS

# Fig. 5

# Fig. 6

# Fig. 7

Fig. 8

Fig. 9

Fig. 10

## Fig. 11

## Fig. 12

$Zs=1.001$

$Zs=1.010$

# Fig. 13

Fig. 14

Fig. 15

# Fig. 16

# Fig. 17

Fig. 18

Fig. 19

Fig. 20

Fig. 21

Fig. 22

## INTERNATIONAL SEARCH REPORT

| International application No. |
|---|
| **PCT/JP2022/032578** |

**A. CLASSIFICATION OF SUBJECT MATTER**

***G01N 33/86*** (2006.01)i

FI:  G01N33/86

According to International Patent Classification (IPC) or to both national classification and IPC

**B. FIELDS SEARCHED**

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2003-169700 A (SYSMEX CORP.) 17 June 2003 (2003-06-17) claims, paragraphs [0013], [0019]-[0035], fig. 4, 5 | 1-3, 5-7, 15-18 |
| X | JP 2007-107889 A (SYSMEX CORP.) 26 April 2007 (2007-04-26) claims, paragraphs [0032], [0038]-[0048], fig. 7 | 1-7, 15, 17-18 |
| A | DOWNEY, C. et al. Novel and diagnostically applicable information from optical waveform analysis of blood coagulation in disseminated intravascular coagulation. British Journal of Haematology. 1997, vol. 98, pp. 68-73 entire text, all drawings | 1–18 |
| A | 松本智子. 凝固波形解析の活用法と見方. Medical Technology. 15 January 2018, vol. 46, no. 1, pp. 66-71, non-official translation (MATSUMOTO, Tomoko. How to utilize and view clot waveform analysis.) entire text, all drawings | 1-18 |
| A | JP 2019-086517 A (SEKISUI MEDICAL CO., LTD.) 06 June 2019 (2019-06-06) entire text, all drawings | 1-18 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 September 2022** | **04 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2022/032578** |

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2010-217059 A (SHIMADZU CORP.) 30 September 2010 (2010-09-30)<br>entire text, all drawings | 1-18 |
| A | JP 2018-072156 A (SYSMEX CORP.) 10 May 2018 (2018-05-10)<br>entire text, all drawings | 1-18 |
| A | WO 2020/158948 A1 (SEKISUI MEDICAL CO., LTD.) 06 August 2020 (2020-08-06)<br>entire text, all drawings | 1-18 |

<div style="text-align: center">

**INTERNATIONAL SEARCH REPORT**
**Information on patent family members**

</div>

| International application No. |
| --- |
| **PCT/JP2022/032578** |

| Patent document cited in search report | | | Publication date (day/month/year) | Patent family member(s) | | | Publication date (day/month/year) |
| --- | --- | --- | --- | --- | --- | --- | --- |
| JP | 2003-169700 | A | 17 June 2003 | US 2003/0138962 A1 claims, paragraphs [0060]-[0086], fig. 4, 5 EP 1316802 A2 | | | |
| JP | 2007-107889 | A | 26 April 2007 | (Family: none) | | | |
| JP | 2019-086517 | A | 06 June 2019 | (Family: none) | | | |
| JP | 2010-217059 | A | 30 September 2010 | (Family: none) | | | |
| JP | 2018-072156 | A | 10 May 2018 | US 2018/0120292 A1 entire text, all drawings EP 3315972 A1 | | | |
| WO | 2020/158948 | A1 | 06 August 2020 | US 2022/0146537 A1 entire text, all drawings EP 3919913 A1 CN 113366319 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003169700 A
- JP 2010217059 A
- JP 2007107889 A
- JP 2020068877 A

- JP 2020048676 W
- JP H6249855 A
- JP 2020039344 A